# EUROPÄISCHE PATENTSCHRIFT

(11) **EP 4 141 774 B1**
(45) Veröffentlichungstag und Bekanntmachung des Hinweises auf die Patenterteilung: **02.07.2025**
(21) Anmeldenummer: 21193678.6
(22) Anmeldetag: 28.08.2021
(51) Int. Cl.: G06Q 30/06, A41H 1/02, A61B 5/107

(54) **VERFAHREN UND SYSTEM ZUM AUSWÄHLEN EINES FAHRRADPRODUKTS**
METHOD AND SYSTEM FOR SELECTING A BICYCLE PRODUCT
PROCÉDÉ ET SYSTÈME DE SÉLECTION D'UN PRODUIT DE VÉLO

(43) Veröffentlichungstag der Anmeldung: 01.03.2023
(73) Patentinhaber: SQlab GmbH, 82024 Taufkirchen (DE)
(72) Erfinder: Hild, Tobias, 82024 Taufkirchen (DE)
(74) Vertreter: Koelle, Alexander

(56) Entgegenhaltungen:
- EP-A1- 2 508 126
- DE-U1- 202006 008 296
- US-A1- 2013 179 288
- US-A1- 2018 374 232
- US-A1- 2019 384 874
- ANONYMOUS: "BikeDynamics - Bike Fitting Specialists - Saddle Height Calculator", 6 March 2020 (2020-03-06), XP055910612, Retrieved from the Internet <URL:https://web.archive.org/web/20200306105220/https://bikedynamics.co.uk/saddleheightformulae.htm> [retrieved on 20220407]

## Beschreibung

### Technisches Gebiet

Die vorliegende Anmeldung betrifft ein Verfahren und ein System zum Auswählen zumindest eines Fahrradsattels aus einer Vielzahl von in einer Datenbank hinterlegten Fahrradsatteln für einen Fahrradfahrer. Das Verfahren bzw. das System umfasst eine Kamera eines Mobilgeräts, durch welche ein Foto von zumindest einem erfassten Gesäßabdruck des Fahrradfahrers aufgenommen wird, und umfasst eine Steuerung, durch welche auf Basis des aufgenommenen Fotos einen Sitzknochenabstand des Fahrradfahrers bestimmt wird, wobei darauf basierend eine Satteleignung einer Mehrzahl von Fahrradsätteln bestimmt wird, wobei darauf basierend eine priorisierte Liste der Mehrzahl von Fahrradsätteln bestimmt wird, und wobei darauf basierend eine Auswahl von zumindest einem Fahrradsattel aus der Mehrzahl von Fahrradsätteln für den Fahrradfahrer durch das Mobilgerät ausgegeben wird.

### Stand der Technik

Bei der Auswahl eines individuell an einen Fahrradfahrer angepassten Fahrradprodukts, wie z.B. Fahrradsattel, Fahrradgriff, Fahrradrahmen, Fahrrad und/oder Fahrradhelm, besteht oftmals der Nachteil, dass entsprechende Körperparameter des Fahrradfahrers durch einen Fachverkäufer zuerst aufwendig gemessen bzw. vermessen werden müssen. Anschließend müssen die gemessenen Körperparameter in Bezug auf das gewünschte Fahrradprodukt manuell ausgewertet und dann einem an den Fahrradfahrer optimal angepassten Fahrradprodukt zugeordnet werden. Ein entsprechender Vorgang ist oftmals nicht nur mit einem hohen Zeit- und damit Kostenaufwand verbunden, sondern weist auch in Abhängigkeit dem Fachverkäufer oftmals nur begrenzt zur Verfügung stehenden Detailinformationen hinsichtlich der relevanten Fahrradprodukten eine hohe Ungenauigkeit bzw. Fehleranfälligkeit auf.

Aus dem Stand der Technik ist es beispielsweise bekannt, dass die Kontur des Gesäßes des Fahrradfahrers, insbesondere der Sitzknochenabstand zwischen den beiden Sitzknochen des Fahrradfahrers, berücksichtigt wird. Eine entsprechende herkömmlich bekannte Erfassung des Sitzknochenabstands erfolgt oftmals durch Druckmessfolien, Messpappen, Messschablonen oder Noppenplatten. Mit Hilfe eines entsprechend manuell erfassten Sitzknochenabstands des Fahrradfahrers war ein Fachverkäufer bisher in der Lage einen für den Fahrradfahrer optimalen Sattel auszuwählen und/oder zu empfehlen.

Hierbei sind im Stand der Technik unterschiedliche Erfassungsmethoden offenbart. Die DE 20 2006 008 296 U1 und EP 2 508 126 A1/B1 offenbaren ein Druckmesssystem zur Ermittlung eines Sitzknochenabstands eines menschlichen Beckens. Die EP 2 703 261 B1 offenbart ein Verfahren zur individuellen Bestimmung einer Fahrrad-Sattelgröße. Die EP 3 290 887 A1 offenbart ein Verfahren und eine Vorrichtung zur Bestimmung einer Fahrrad-Sattelgröße. Die EP 3 395 658 A1 offenbart ein Verfahren zur individuellen Bestimmung einer Fahrrad-Sattelgröße.

Ein Nachteil entsprechender herkömmlicher Sitzknochenabstandserfassungssysteme ist unter anderem, dass zum Teil teure und komplexe Apparaturen benötigt werden, dass die Auswertung der entsprechend erfassten Gesäßabdrücke durch den Fachverkäufer oftmals aufwendig ist, dass die Gesäßabdrücke bzw. Sitzknochenabstände oftmals nicht oder nur unzureichend in digitalisierter Form gespeichert werden können, und dass die Zuordnung der Gesäßabdrücke bzw. Sitzknochenabstände, zu entsprechenden für den Fahrradfahrer optimalen Fahrradsätteln manuell durch den Fachverkäufer erfolgt und dadurch fehleranfällig sein kann.

Eine einfache und automatisierte Erfassung von individuellen Körperparametern eines Fahrradfahrers, sowie eine entsprechende darauf basierende einfache und automatisierte Bestimmung von Produkteignungen entsprechender Fahrradprodukte, sowie die sich daran anschließende Ausgabe einer Auswahl von einer für den jeweiligen Fahrradfahrer optimalen Auswahl von Fahrradprodukten ist aus dem Stand der Technik nicht bekannt.

Insbesondere ist aus dem Stand der Technik nicht bekannt, dass eine einfache und automatisierte Erfassung von Körperparametern eines Fahrradfahrers durch ein Mobilgerät bzw. durch eine auf dem Mobilgerät ausgeführte App, durchgeführt werden kann.

Auch ist aus dem Stand der Technik nicht bekannt, dass eine durch ein Mobilgerät durchgeführte einfache und automatisierte Erfassung, zu einer für den Fahrradfahrer optimalen Auswahl von Fahrradprodukten führen kann.

In der EP 2 535 248 B1 ist lediglich die Körperparameter-abhängige Ausgabe eines Fahrradmodels offenbart. In der US 2002 / 180 166 A1 ist lediglich die sensorische Erfassung einer Fahrposition eines Fahrradfahrers offenbart. In der US 9,019,349 B2 ist lediglich ein Motion-Capture-Verfahren offenbart. In der US 9,381,417 B2 ist lediglich eine automatische Erfassung einer Fahrposition eines Fahrradfahrers offenbart.

In der US 2019/0384874 A1 ist ein System zur Echtzeit-Anpassung von Fahrradgrößeninformationen für einen Fahrradfahrer offenbart. In der EP 2 508 126 A1 ist ein Druckmesssystem zur Ermittlung des Sitzknochenabstands eines menschlichen Beckens offenbart.

### Offenlegung der Anmeldung

Die vorliegende Anmeldung stellt sich zur Aufgabe, ein Verfahren und ein System zur automatisierten und individualisierten Auswahl eines Fahrradsattels, für einen Fahrradfahrer bereitzustellen.

Die Aufgabe der Anmeldung wird gemäß einem ersten Aspekt durch ein Verfahren zum Auswählen zumindest eines Fahrradsattels für einen Fahrradfahrer nach dem unabhängigen Anspruch 1, sowie gemäß einem zweiten Aspekt durch ein System zum Auswählen zumindest eines Fahrradsattels für einen Fahrradfahrer nach dem unabhängigen Anspruch 6 gelöst. Die weiteren abhängigen Ansprüche beanspruchen bevorzugte Ausführungsformen.

Gemäß einem ersten Aspekt der vorliegenden Anmeldung wird die vorliegende Aufgabe durch ein Verfahren zum Auswählen zumindest eines Fahrradprodukts aus einer Vielzahl von in einer Datenbank hinterlegten Fahrradprodukten für einen Fahrradfahrer gelöst, umfassend die folgenden Verfahrensschritte: Eingeben von zumindest einem Eingabeparameter, insbesondere Geschlecht, Fahrradtyp, Fahrhäufigkeit des Fahrradfahrers, Körpergewicht des Fahrradfahrers, Sitzposition des Fahrradfahrers, anatomische Besonderheiten des Fahrradfahrers, Körpergröße des Fahrradfahrers, Beinstellung des Fahrradfahrers, Fußtyp des Fahrradfahrers, Schuhgröße des Fahrradfahrers, Fußstellung des Fahrradfahrers, und/oder eine übliche Dauer einer Fahrradfahrt des Fahrradfahrers, durch eine Eingabevorrichtung eines Mobilgeräts, Aufnehmen eines Fotos von zumindest einem Körperteil des Fahrradfahrers durch eine Kamera des Mobilgeräts, Bestimmen zumindest eines Körperparameters des Fahrradfahrers auf Basis des aufgenommenen Fotos durch eine Steuerung, Bestimmen jeweils einer Produkteignung einer Mehrzahl von Fahrradprodukten der Vielzahl von in der Datenbank hinterlegten Fahrradprodukten in Abhängigkeit des bestimmten Körperparameters und in Abhängigkeit des zumindest einen eingegebenen Eingabeparameters durch die Steuerung, Bestimmen einer priorisierten Liste der Mehrzahl von Fahrradprodukten durch die Steuerung, wobei in der priorisierten Liste die Priorität der jeweiligen Fahrradprodukte mit zunehmender Produkteignung steigt, und Ausgeben einer Auswahl von zumindest einem Fahrradprodukt für den Fahrradfahrer durch das Mobilgerät, wobei die Auswahl das zumindest eine Fahrradprodukt der priorisierten Liste der Mehrzahl von Fahrradprodukten umfasst, dessen jeweilige bestimmte Produkteignung die geringste Differenz oder gar keine Differenz zu einer maximalen Produkteignung aufweist.

Durch die Steuerung, insbesondere in Kombination mit einem auf dem Mobilgerät ablaufendem Programm bzw. App, kann somit auf eine einfache und automatisierte Weise eine Auswahl von individuell an den Fahrradfahrer angepassten Fahrradprodukten auf dem Mobilgerät ausgegeben werden, z.B. auf einem Display des Mobilgeräts.

Durch die Eingabevorrichtung des Mobilgeräts, insbesondere ein Touch-Display und/oder Touch-Button des Mobilgeräts, kann der Nutzer des Mobilgeräts zumindest einen der zur Fahrradproduktbestimmung relevanten Eingabeparameter eingeben. Der zumindest eine Eingabeparameter umfasst insbesondere einen einzigen oder eine Mehrzahl der folgenden Eingabeparameter, Geschlecht, Fahrradtyp, Fahrhäufigkeit des Fahrradfahrers, Körpergewicht des Fahrradfahrers, Sitzposition des Fahrradfahrers, anatomische Besonderheiten des Fahrradfahrers, Körpergröße des Fahrradfahrers, Beinstellung des Fahrradfahrers, Fußtyp des Fahrradfahrers, Schuhgröße des Fahrradfahrers, Fußstellung des Fahrradfahrers, und/oder eine übliche Dauer einer Fahrradfahrt des Fahrradfahrers.

Insbesondere umfasst der Eingabeparameter Fahrradtyp die weitere Unterauswahl, ob ein E-Bike gewünscht ist oder nicht.

Je nach gewünschtem Fahrradprodukt kann auch nur eine Unterauswahl der insbesondere genannten Eingabeparameter durch die Eingabevorrichtung des Mobilgeräts eingegeben werden, da z.B. die Beinstellung des Fahrradfahrers bei der Auswahl eines individuell an den Fahrradfahrer angepassten Fahrradhelms unter Umständen irrelevant sein kann.

Insbesondere umfasst das Eingeben des zumindest einen Eingabeparameters das Eingeben einer Mehrzahl von Eingabeparametern, wobei insbesondere die Reihenfolge der Eingaben der Eingabeparameter veränderbar ist.

Nach dem Eingeben kann der zumindest eine Eingabeparameter insbesondere hinterlegt werden, um z.B. bei einer späteren Fahrradproduktauswahl für denselben Fahrradfahrer einen raschen Zugriff auf zumindest einige der vormals eingegebenen Eingabeparameter zu ermöglichen.

Anschließend wird ein Foto von zumindest einem Körperteil des Fahrradfahrers durch eine Kamera des Mobilgeräts aufgenommen. Insbesondere kann das zumindest eine Körperteil ein einziges Körperteil des Fahrradfahrers umfassen, so dass z.B. für die Auswahl eines Fahrradhelms ein Foto des Kopfes des Fahrradfahrers aufgenommen, bzw. so dass z.B. für die Auswahl eines Fahrradgriffs ein Foto einer Hand des Fahrradfahrers aufgenommen wird.

Es können jedoch auch eine Mehrzahl von Körperteilen, insbesondere auch der gesamte Körper des Fahrradfahrers durch die Kamera des Mobilgeräts erfasst werden, so dass z.B. auf Basis der fotografierten Körperteile, bzw. des fotografierten gesamten Körpers des Fahrradfahrers unterschiedlichen Fahrradprodukte ausgegeben werden können.

Es kann jedoch auch ein Abbild zumindest eines Körperteils durch die Kamera des Mobilgeräts fotografiert werden, wie z.B. einen Gesäßabdruck eines Gesäßes des Fahrradfahrers, um auf dessen Basis eine vorteilhafte Ausgabe von Fahrradprodukten, z.B. Fahrradsätteln zu ermöglichen.

Das durch die Kamera aufgenommene Foto des zumindest einen Körperteils des Fahrradfahrers kann ein einziges Foto oder eine Mehrzahl von Fotos umfassen, auf dessen Basis oder auf deren Basis anschließend der zumindest eine Körperparameter des Fahrradfahrers bestimmt werden kann.

Der zumindest eine Körperparameter des Fahrradfahrers wird auf Basis des durch die Kamera des Mobilgeräts aufgenommenen Fotos durch eine Steuerung bestimmt.

Die Steuerung kann die Steuerung des Mobilgeräts, insbesondere den Prozessor des Mobilgeräts, umfassen.

Alternativ kann die Steuerung Teil einer externen Datenverarbeitungseinrichtung sein, welche den zumindest einen Körperparameter des Fahrradfahrers auf Basis des durch die Kamera des Mobilgeräts aufgenommenen Fotos bestimmt. Hierbei weist das Mobilgerät insbesondere eine erste Kommunikationseinrichtung auf, und weist die externe Datenverarbeitungseinrichtung insbesondere eine zweite Kommunikationseinrichtung auf, so dass Daten von dem Mobilgerät, insbesondere das aufgenommene Foto, durch die erste Kommunikationseinrichtung und durch die zweite Kommunikationseinrichtung der externen Datenverarbeitungseinrichtung zur Verfügung gestellt werden können.

Der zumindest eine durch die Steuerung bestimmte Körperparameter umfasst insbesondere einen einzigen Körperparameter oder eine Mehrzahl von Körperparametern.

Je nach dem ausgewählten Fahrradprodukt können unterschiedliche Körperparameter bestimmt werden. Soll beispielsweise eine Auswahl von Fahrradsätteln ausgegeben werden, wird insbesondere der Sitzknochenabstand zwischen den beiden Sitzknochen des Gesäßes des Fahrradfahrers als relevanter Körperparameter bestimmt. Soll beispielsweise eine Auswahl von Fahrradgriffen ausgegeben werden, wird insbesondere eine Handbreite und/oder eine Handlänge einer Hand des Fahrradfahrers als relevanter Körperparameter bestimmt. Soll beispielsweise eine Auswahl von Fahrradrahmen ausgegeben werden, wird insbesondere eine Schrittlänge des Fahrradfahrers als relevanter Körperparameter bestimmt. Soll beispielsweise eine Auswahl von Fahrradhelmen ausgegeben werden, wird insbesondere ein Kopfumfang eines Kopfes des Fahrradfahrers als relevanter Körperparameter bestimmt.

Anschließend wird jeweils eine Produkteignung einer Mehrzahl von Fahrradprodukten der Vielzahl von in der Datenbank hinterlegten Fahrradprodukten in Abhängigkeit des bestimmten zumindest einen Körperparameters und in Abhängigkeit des zumindest einen eingegebenen Eingabeparameters durch die Steuerung bestimmt.

Hier kann die Datenbank und die Steuerung insbesondere Teil einer externen Datenverarbeitungseinrichtung sein, durch welche das entsprechende Bestimmen der jeweiligen Produkteignung durchgeführt wird, wobei hierbei insbesondere ein Datenaustausch zwischen dem Mobilgerät und der externen Datenverarbeitungseinrichtung durch eine erste Kommunikationseinrichtung des Mobilgeräts und durch eine zweite Kommunikationseinrichtung der externen Datenverarbeitungseinrichtung durchgeführt wird.

Alternativ kann jedoch die Datenbank und die Steuerung insbesondere Teil des Mobilgeräts sein.

Die entsprechende durch die Steuerung berücksichtigte Mehrzahl von Fahrradprodukten kann insbesondere die gesamte Vielzahl von in der Datenbank hinterlegten Fahrradprodukten umfassen. Alternativ kann die entsprechende durch die Steuerung berücksichtigte Mehrzahl von Fahrradprodukten nur eine Unterauswahl der Vielzahl von in der Datenbank hinterlegten Fahrradprodukten umfassen.

Das Bestimmen der jeweiligen Produkteignung wird in Abhängigkeit von dem eingegebenen zumindest einen Körperparameter und von dem zumindest einen eingegebenen Eingabeparameter durchgeführt, so dass die bestimmte Produkteignung die individuellen Charakteristika des Fahrradfahrers berücksichtigt.

Beispielsweise umfasst die bestimmte jeweilige Produkteignung einen numerischen Wert, z.B. 9, oder eine Prozentangabe, z.B. 90%.

Anschließend bestimmt die Steuerung eine priorisierte Liste der Mehrzahl von Fahrradprodukten, wobei in der priorisierten Liste die Priorität der jeweiligen Fahrradprodukte mit zunehmender Produkteignung steigt.

Durch die priorisierte Liste wird somit ein vorteilhaftes Ranking der Mehrzahl von Fahrradprodukten mit zunehmender Produkteignung erstellt. Umfasst die Mehrzahl von Fahrradprodukten beispielsweise drei Fahrradprodukte mit jeweiligen Produkteignungen von 80%, 90% und 95%, so weist das Fahrradprodukt mit einer Produkteignung von 95% die höchste Priorität auf, weist das Fahrradprodukt mit einer Produkteignung von 90% die zweithöchste Priorität auf, und weist das Fahrradprodukt mit einer Produkteignung von 80% die dritthöchste Priorität auf.

Anschließend wird durch das Mobilgerät eine Auswahl von zumindest einem Fahrradprodukt, insbesondere ein einziges Fahrradprodukt oder mehrere Fahrradprodukte der priorisierten Liste der Mehrzahl von Fahrradprodukten durch das Mobilgerät ausgegeben. Die Auswahl umfasst das Fahrradprodukt oder die Fahrradprodukte dessen bestimmte jeweilige Produkteignung die geringste Differenz oder gar keine Differenz zu einer maximalen Produkteignung aufweist.

Insbesondere entspricht die maximale Produkteignung 100%, und es wird das zumindest eine Fahrradprodukt ausgegeben, dessen jeweilige Produkteignung die geringste Differenz oder gar keine Differenz zu der Prozentangabe von 100% aufweist.

Insbesondere umfasst das Ausgeben das Ausgeben einer Auswahl von mehreren Fahrradprodukten für den Fahrradfahrer durch das Mobilgerät, wobei die Auswahl die mehreren Fahrradprodukte der priorisierten Liste der Mehrzahl von Fahrradprodukten umfasst, deren jeweilige Differenz zwischen der jeweiligen Produkteignung und der maximalen Produkteignung geringer als ein Schwellenwert ist. Insbesondere umfasst der Schwellenwert insbesondere einen Wert von 10%, 5 %, 2%, 1% oder beliebige andere Werte.

Somit kann aus der Vielzahl von in der Datenbank hinterlegten Fahrradprodukten durch das Mobilgerät anhand der jeweiligen bestimmten Produkteignung eine spezifische Unterauswahl von zumindest einem Fahrradprodukt für den Fahrradfahrer ausgegeben werden.

Das Ausgeben der Auswahl des zumindest einen Fahrradprodukts umfasst insbesondere das Darstellen der Auswahl auf einem Display des Mobilgeräts.

Ein Mobilgerät im Sinne der vorliegenden Anmeldung umfasst hierbei insbesondere ein tragbares elektronisches Gerät, insbesondere ein Smartphone, ein Smart-Pad, eine Smartwatch, ein Notebook oder dergleichen.

Somit kann durch das Mobilgerät in Kombination mit der Steuerung eine automatisierte und individualisierte Auswahl von zumindest einem Fahrradprodukt für den Fahrradfahrer bereitgestellt werden.

Aufgrund der geringen Größe des Mobilgeräts und der in das Mobilgerät integrierten Kamera kann die Aufnahme des Körperteils und die darauf basierende Ausgabe der Auswahl des zumindest einen Fahrradprodukts einfach, schnell und für den Fahrradfahrer mit einem besonders geringen Kostenaufwand, sowie durch die Berücksichtigung der Vielzahl von in der Datenbank hinterlegten Fahrradprodukten durch die Steuerung mit einer geringen Fehleranfälligkeit erfolgen.

Erfindungsgemäß umfasst das Verfahren das Auswählen zumindest eines Fahrradsattels aus einer Vielzahl von in einer Datenbank hinterlegten Fahrradsätteln für einen Fahrradfahrer, wobei das Eingeben des zumindest einen Eingabeparameters insbesondere das Eingeben von Geschlecht, Fahrradtyp, Fahrhäufigkeit des Fahrradfahrers, Körpergewicht des Fahrradfahrers, Sitzposition des Fahrradfahrers, anatomische Besonderheiten des Fahrradfahrers, und/oder eine übliche Dauer einer Fahrradfahrt des Fahrradfahrers umfasst, wobei das Aufnehmen des Fotos das Erfassen eines Gesäßabdrucks des Fahrradfahrers durch eine Erfassungsvorrichtung umfasst, wobei der Gesäßabdruck jeweils einen Druckbereich der beiden Sitzknochen des auf der Erfassungsvorrichtung sitzenden Fahrradfahrers umfasst und das Aufnehmen eines Fotos des erfassten Gesäßabdrucks durch die Kamera des Mobilgeräts umfasst, wobei das Bestimmen des zumindest einen Körperparameters des Fahrradfahrers das Bestimmen eines Sitzknochenabstands des Fahrradfahrers auf Basis des aufgenommenen Fotos des erfassten Gesäßabdrucks durch die Steuerung umfasst, wobei das Bestimmen der jeweiligen Produkteignung das Bestimmen jeweils einer Satteleignung einer Mehrzahl von Fahrradsätteln der Vielzahl von in der Datenbank hinterlegten Fahrradsätteln in Abhängigkeit des bestimmten Sitzknochenabstands und in Abhängigkeit des zumindest einen eingegebenen Eingabeparameters durch die Steuerung umfasst, wobei das Bestimmen der priorisierten Liste das Bestimmen einer priorisierten Liste der Mehrzahl von Fahrradsätteln durch die Steuerung umfasst, wobei in der priorisierten Liste die Priorität der jeweiligen Fahrradsättel mit zunehmender Satteleignung steigt, und wobei das Ausgeben der Auswahl das Ausgeben von zumindest einem Fahrradsattel für den Fahrradfahrer durch das Mobilgerät umfasst, wobei die Auswahl den zumindest einen Fahrradsatteln der priorisierten Liste der Mehrzahl von Fahrradsatteln umfasst, dessen jeweilige bestimmte Satteleignung die geringste Differenz oder gar keine Differenz zu einer maximalen Satteleignung aufweist. Hierbei sitzt der Fahrradfahrer zuerst auf der Erfassungsvorrichtung um den Gesäßabdruck des Fahrradfahrers zu erhalten. Der Gesäßabdruck umfasst jeweils einen Druckbereich der beiden Sitzknochen des auf der Erfassungsvorrichtung sitzenden Fahrradfahrers.

Das durch die Kamera des Mobilgeräts aufgenommene Foto des Gesäßabdrucks wird anschließend durch die Steuerung, insbesondere in Kombination mit einem auf dem Mobilgerät installierten Programm bzw. App, ausgewertet, um auf Basis des aufgenommenen Fotos des Gesäßabdrucks den Sitzknochenabstand des Fahrradfahrers zu bestimmen.

Um zu einer personalisierten, d.h. individualisierten Auswahl von zumindest einem Fahrradsattel für den Fahrradfahrer zu gelangen, muss die Steuerung noch über zumindest einen in der Steuerung hinterlegten Eingabeparameter, welcher Geschlecht, Fahrradtyp, Fahrhäufigkeit des Fahrradfahrers, Körpergewicht des Fahrradfahrers, Sitzposition des Fahrradfahrers, anatomische Besonderheiten des Fahrradfahrers, Körpergröße des Fahrradfahrers, und/oder eine übliche Dauer einer Fahrradfahrt des Fahrradfahrers umfasst, verfügen.

Der zumindest eine Eingabeparameter wird hierbei direkt durch die Eingabevorrichtung, insbesondere ein Touch-Display und/oder Touch-Button des Mobilgeräts, eingegeben, um den zumindest einen Eingabeparameter der Steuerung zur Verfügung zu stellen.

In Abhängigkeit des zumindest einen Eingabeparameters und des bestimmten Sitzknochenabstands bestimmt die Steuerung die jeweilige Satteleignung, insbesondere als einen numerischen Wert oder als eine Prozentangabe, wobei anschließend eine priorisierte Liste mit aufsteigender Satteleignung bestimmt wird.

Anschließend gibt das Mobilgerät zumindest einen Fahrradsattel für den Fahrradfahrer aus, dessen jeweilige bestimmte Satteleignung die geringste Differenz oder gar keine Differenz zu einer maximalen Satteleignung, insbesondere 100%, aufweist, insbesondere durch eine graphische Übersicht des zumindest einen ausgewählten Fahrradsattels auf einer Ausgabevorrichtung, insbesondere Display, des Mobilgeräts.

In einer Ausführungsform umfasst die Erfassungsvorrichtung eine Grundplatte mit Noppen, auf welchen ein Blatt Papier angeordnet ist, wobei der Fahrradfahrer während des Erfassens des Gesäßabdrucks mit seinem Gesäß auf dem Papier sitzt, und wobei während des Erfassens des Gesäßabdrucks die Noppen durch das Papier hindurchgedrückt werden, um die Druckbereiche der beiden Sitzknochen des Fahrradfahrers auf dem Papier zu erhalten.

Dadurch wird der technische Vorteil erreicht, dass eine einfache, schnell durchzuführende und für den Fahrradfahrer nicht unangenehme Erfassung des Gesäßbereichs erreicht wird. Hierbei wird insbesondere sichergestellt, dass spezifisch in dem Bereich des Papiers, auf dem der Fahrradfahrer mit den beiden Sitzknochen sitzt, die Noppen durch das Papier hindurchgedrückt werden. Insbesondere in den anderen Bereichen des Papiers, welche nicht in Kontakt mit dem Gesäß des Fahrradfahrers kommen, wird sichergestellt, dass die Noppen der Grundplatte nicht durch das Papier durchgedrückt werden. Diese Vorgehensweise und Verwendung ist im Detail u.a. in EP 2 508 126 A1/B1 erläutert, dargestellt und offenbart.

Wird das Papier anschließend von der Grundplatte mit den Noppen entfernt, ist der Gesäßabdruck des Fahrradfahrers visuell auf dem Papier sichtbar und durch die Kamera des Mobilgeräts kann vorteilhaft ein Foto des Gesäßabdrucks aufgenommen und durch die Steuerung anschließend weiter ausgewertet werden.

In einer Ausführungsform ist auf dem Blatt Papier ein umlaufender Rahmen angeordnet, und umfasst das Bestimmen des Sitzknochenabstands das Erkennen und Rektifizieren sowie Ausrichten des aufgenommenen Fotos des Gesäßabdrucks auf Basis des umlaufenden Rahmens durch die Steuerung.

Dadurch wird der technische Vorteil erreicht, dass der auf dem Blatt Papier angeordnete umlaufende Rahmen sicherstellt, dass vor dem Auswerten des Fotos des Gesäßabdrucks das aufgenommene Foto durch die Steuerung wirksam ausgerichtet und die Größe kalibriert werden kann. Der umlaufende Rahmen dient ferner der Kalibrierung der Größenverhältnisse des aufgenommenen Fotos und insbesondere des Gesäßabdrucks. Der umlaufende Rahmen erlaubt der Steuerung insbesondere den Bereich des Papiers innerhalb des Rahmens von dem Bereich des Papiers außerhalb des Rahmens abzugrenzen, um beispielweise außerhalb des Rahmens in dem Foto angeordnete Objekte bei der Auswertung des Fotos auszuschließen. Der umlaufende Rahmen dient als Referenzhintergrund mit einer definierten Größe. Mittels des umlaufenden Rahmens kann die Steuerung den Gesäßabdruck und den ermittelten Sitzknochenabstand zu der definierten Größe des umlaufenden Rahmens in Bezug setzen, um dadurch die genaue Abmessung des Sitzknochenabstands präzise zu bestimmen.

In einer Ausführungsform umfasst die Erfassungsvorrichtung ein lichttransparentes mit Gel gefülltes Sitzkissen, wobei der Fahrradfahrer während des Erfassens des Gesäßabdrucks mit seinem Gesäß auf dem Sitzkissen sitzt, und wobei während des Erfassens des Gesäßabdrucks sich die Verteilung des Gels innerhalb des Sitzkissens verändert, um eine visuelle Darstellung der Druckbereiche der beiden Sitzknochen des Fahrradfahrers auf dem Sitzkissen zu erhalten. Diese Vorgehensweise und Verwendung ist im Detail u.a. in DE 20 2006 008 296 U1 erläutert, dargestellt und offenbart.

Dadurch wird der technische Vorteil erreicht, dass nach dem Aufstehen des Fahrradfahrers von der Erfassungseinrichtung, durch die Sitzknochen des Fahrradfahrers eine dem Gesäßabdruck des Fahrradfahrers entsprechende und auf der Änderung der Verteilung des Gels in dem Sitzkissen basierende Vertiefung an der Oberfläche des Sitzkissens erhalten wird. Die dem Gesäßabdruck entsprechende Vertiefung an der Oberfläche des Sitzkissens kann visuell erfasst werden, und ist auch auf dem durch die Kamera des Mobilgeräts aufgenommenen Foto des Sitzkissens sichtbar und kann durch die Steuerung zur Bestimmung des Sitzknochenabstands ausgewertet werden.

In einer Ausführungsform umfasst das Bestimmen des Sitzknochenabstands des Fahrradfahrers die folgenden Verfahrensschritte: Bestimmen eines ersten Mittelpunkts eines in dem aufgenommenen Foto des erfassten Gesäßabdrucks visuell dargestellten ersten Druckbereichs eines ersten Sitzknochens des Fahrradfahrers durch die Steuerung, Bestimmen eines zweiten Mittelpunkts eines in dem aufgenommenen Foto des erfassten Gesäßabdrucks visuell dargestellten zweiten Druckbereichs eines zweiten Sitzknochens des Fahrradfahrers durch die Steuerung, und Bestimmen eines Abstands zwischen dem ersten und zweiten Mittelpunkt durch die Steuerung, um den Sitzknochenabstand des Fahrradfahrers zu bestimmen.

Dadurch wird der technische Vorteil erreicht, dass eine wirksame Bestimmung des Sitzknochenabstands des Fahrradfahrers erreicht wird.

Insbesondere wird die Bestimmung des Sitzknochenabstands durch eine Steuerung einer externen Datenverarbeitungseinrichtung oder durch eine Steuerung des Mobilgeräts durchgeführt.

Insbesondere wird zur Auswertung des Fotos des Gesäßabdrucks das Bildauswertungsverfahren der "Blob-Detektion" verwendet. Als sog. "Blobs" werden runde Objekte bezeichnet. Blobs sind insbesondere die Durchstoßpunkte der Noppen durch das Papier. Durch den Schattenwurf erscheinen diese als dunkle runde Kreise, sogenannte Blobs. Die Blobs ergeben zusammen den Gesäßabdruck der beiden Sitzknochen. Lediglich exemplarisch wird auf die folgenden Verfahren zur "Blob-Detektion" verwiesen, https://docs.opencv.org/3.4/dO/d7a/classcv 1 1 SimpleBlobDetector.html und https://ieeexplore.ieee.org/document/7467122.

Insbesondere wird hierbei ein "Gaussian Mixture Model" mit zwei Zentroiden über die Blobs bzw. die runden Objekte gelegt, und die Blobs bzw. die runden Objekte werden hierbei entsprechend ihrer Größe gewichtet. Insbesondere weisen die Zentroide jeweils einen Mittelwert und eine Varianz auf. Als Referenz wird hierbei verwiesen auf: Bishop, C., Pattern Recognition and Machine Learning (Information Science and Statistics), Springer, 1. Auflage 2007, Seite 423 ff. und http:/www.cse.psu.edu/-rte12/CSE586Spring2010/papers/prmIMixturesEM.pdf.

Um Ausreißer zu umgehen, wird insbesondere die Auftrittswahrscheinlichkeit eines jeden runden Objekts zu den gegebenen Zentroiden berechnet. Ist diese unter einem Schwellwert, wird dieses runde Objekt durch die Steuerung nicht weiter berücksichtigt. Nach diesem Schritt werden die Zentroide insbesondere ein zweites Mal über die noch vorhandenen runden Objekte gelegt.

Der Abstand der beiden Mittelwerte der Zentroide entspricht dem Abstand zwischen dem ersten und zweiten Mittelpunkt in der Bild-Ebene in der Einheit Pixel. Da die Abmessungen des Rechtecks auf dem Bild bekannt sind, kann der Abstand in der Bild Ebene in einen realen Abstand mit der Einheit cm übersetzt werden, um den Sitzknochenabstand zu erhalten.

Erfindungsgemäß umfasst das jeweilige Bestimmen der Satteleignung die folgenden Verfahrensschritte: Zuordnen jeweils eines numerischen Wertes zu dem bestimmten Sitzknochenabstand und zu dem zumindest einen Eingabeparameter durch die Steuerung, Gewichten der jeweils zugeordneten numerischen Werte durch die Steuerung, um gewichtete numerische Werte zu erhalten, und Bilden eines Mittelwerts aus der Summe der gewichteten numerischen Werte, um die jeweilige Satteleignung zu erhalten.

Dadurch wird der technische Vorteil erreicht, dass durch die Verwendung von numerischen Werten die jeweilige Satteleignung durch die Steuerung einfach und mit einer geringen Rechenleistung ermittelt werden kann.

Insbesondere umfassen die numerischen Werte, welche zu dem bestimmten Sitzknochenabstand und zu dem zumindest einen Eingabeparameter durch die Steuerung zugeordnet werden, numerische Werte in einem Bereich zwischen 0 und 100. Hierbei steht der untere Schwellenwert 0 des Bereichs für eine geringe Relevanz hinsichtlich der jeweiligen Satteleignung. Hierbei steht der obere Schwellenwert 100 des Bereichs für eine große Relevanz hinsichtlich der jeweiligen Satteleignung.

Das Gewichten der jeweils zugeordneten numerischen Werte durch die Steuerung berücksichtigt hierbei spezifische numerischen Werte dahingehend als besonders, dass ihnen bei der Bestimmung der jeweiligen Satteleignung eine besonders große Relevanz eingeräumt wird.

Insbesondere umfasst der zumindest eine Eingabeparameter die Sattelbreite des Fahrradsattels und den Fahrradtyp, wobei insbesondere der Gewichtungsfaktor bei der Gewichtung des numerischen Wertes, welcher der Sattelbreite des Fahrradsattels zugeordnet ist, und der Gewichtungsfaktor bei der Gewichtung des numerischen Wertes, welcher dem Fahrradtyp des Fahrradsattels zugeordnet ist, zwei beträgt. Insbesondere kann die Auswahl des Fahrradtyps die Unterauswahl umfassen, ob ein E-Bike gewünscht ist, im Rahmen einer "Ja" oder "Nein" Beantwortung, wobei hierbei insbesondere der Gewichtungsfaktor bei der Gewichtung des numerischen Wertes, welcher der E-Bike Auswahl zugeordnet ist, eins beträgt.

Insbesondere umfasst der zumindest eine Eingabeparameter ferner eine Fahrhäufigkeit des Fahrradfahrers, eine übliche Dauer einer Fahrradfahrt des Fahrradfahrers, ein Körpergewicht des Fahrradfahrers, das Geschlecht des Fahrradfahrers, und/oder anatomische Besonderheiten des Fahrradfahrers, wie z.B. Taubheitsgefühle, Sitzknochen, Steißbein, Schambein (bei einer Frau), bzw. Prostata (bei einem Mann), und/oder unterer Rücken, wobei insbesondere der Gewichtungsfaktor bei der Gewichtung des jeweiligen numerischen Wertes, welcher dem jeweiligen Eingabeparameter zugeordnet ist, eins beträgt.

Wenn beispielsweise die gewünschte Sattelbreite des Fahrradsattels mit einem numerischen Wert von 100 hinterlegt ist, und eine Gewichtung mit dem Gewichtungsfaktor zwei stattfindet, resultiert daraus ein gewichteter numerischer Wert von 200, welcher somit die resultierende jeweilige Satteleignung prägt.

Insbesondere kann hierbei bei der Auswahl der anatomischen Besonderheiten des Fahrradfahrers eine mehrfache Unterauswahl getroffen werden, wenn mehrere anatomische Besonderheiten vorliegen, wobei hierbei insbesondere jede Unterauswahl bei der Gewichtung mit einem Gewichtungsfaktor von eins berücksichtigt wird.

Anschließend wird durch die Steuerung ein Mittelwert aus der Summe der gewichteten numerischen Werte gebildet, um die jeweilige Satteleignung zu bestimmen.

Gemäß einem zweiten Aspekt der vorliegenden Anmeldung wird die vorliegende Aufgabe durch ein System zum Auswählen zumindest eines Fahrradsattels aus einer Vielzahl von in einer Datenbank hinterlegten Fahrradsätteln für einen Fahrradfahrer gelöst, umfassend eine Eingabevorrichtung eines Mobilgeräts, welche zum Eingeben von zumindest einem Eingabeparameter, welcher Geschlecht, Fahrradtyp, Fahrhäufigkeit des Fahrradfahrers, Körpergewicht des Fahrradfahrers, Sitzposition des Fahrradfahrers, anatomische Besonderheiten des Fahrradfahrers, Körpergröße des Fahrradfahrers, und/oder eine übliche Dauer einer Fahrradfahrt des Fahrradfahrers umfasst, ausgebildet ist, eine Erfassungsvorrichtung, welche ausgebildet ist, einen Gesäßabdruck des Fahrradfahrers zu erfassen, wobei der Gesäßabdruck jeweils einen Druckbereich der beiden Sitzknochen des auf der Erfassungsvorrichtung sitzenden Fahrradfahrers umfasst, eine Kamera des Mobilgeräts, welche ausgebildet ist, ein Foto des erfassten Gesäßabdrucks des Fahrradfahrers aufzunehmen, und eine Steuerung, welche ausgebildet ist, auf Basis des aufgenommenen Fotos einen Sitzknochenabstand des Fahrradfahrers zu bestimmen, wobei die Steuerung ausgebildet ist, jeweils eine Satteleignung einer Mehrzahl von Fahrradsätteln der Vielzahl von in der Datenbank hinterlegten Fahrradsätteln in Abhängigkeit des bestimmten Sitzknochenabstands und in Abhängigkeit des zumindest einen Eingabeparameters zu bestimmen, wobei die Steuerung ausgebildet ist, jeweils einen numerischen Wert zu dem bestimmten Sitzknochenabstand, und zu dem zumindest einen Eingabeparameter zuzuordnen, wobei die Steuerung ausgebildet ist, die jeweils zugeordneten numerischen Werte zu gewichten, um gewichtete numerische Werte zu erhalten, wobei die Steuerung ausgebildet ist, einen Mittelwert aus der Summe der gewichteten numerischen Werte zu bilden, um die jeweilige Satteleignung zu erhalten, wobei die Steuerung ausgebildet ist, eine priorisierten Liste der Mehrzahl von Fahrradsätteln zu bestimmen, wobei in der priorisierten Liste die Priorität der jeweiligen Fahrradsättel mit zunehmender Satteleignung steigt, wobei das Mobilgerät ausgebildet ist, eine Auswahl von zumindest einem Fahrradsattel für den Fahrradfahrer auszugeben, wobei die Auswahl das zumindest einen Fahrradsattel der Mehrzahl von Fahrradsäteln umfasst, dessen jeweilige bestimmte Satteleignung die geringste Differenz oder gar keine Differenz zu einer maximalen Satteleignung aufweist.

Dadurch wird der technische Vorteil erreicht, dass durch den Nutzer des Mobilgeräts eine besonders einfache und vorteilhafte visuelle Erfassung der Auswahl des Fahrradsattels sichergestellt wird.

Die für das Verfahren gemäß dem ersten Aspekt genannten Ausführungsformen sind ebenso Ausführungsformen für das System gemäß dem zweiten Aspekt.

### Kurze Beschreibung der Figuren

Die Anmeldung wird nun unter Bezugnahme auf die beigefügten Figuren erläutert, die exemplarische und nicht beschränkende Ausführungsformen der Anmeldung zeigen, wobei
- Figur 1: ein System zum Auswählen zumindest eines Fahrradsattels für einen Fahrradfahrer gemäß einer Ausführungsform in einer schematischen Darstellung,
- Figur 2: einen durch eine Erfassungsvorrichtung erfassten Gesäßabdruck eines Fahrradfahrers gemäß einer Ausführungsform,
- Figur 3: eine Bestimmung eines Sitzknochenabstands eines Fahrradfahrers durch eine Steuerung gemäß einer Ausführungsform,
- Figur 4: eine schematische Darstellung einer durch eine Steuerung bestimmten Produkteignung, insbesondere Rahmeneignung, eines Fahrradprodukts, insbesondere Fahrradrahmens, gemäß einer nicht erfindungsgemäßen Ausführungsform,
- Figur 5: eine schematische Darstellung einer durch eine Steuerung bestimmten Auswahl von zumindest einem Fahrradprodukt, insbesondere Fahrradsattel, gemäß einer Ausführungsform,
- Figur 6: eine schematische Darstellung einer Bestimmung einer Handlänge und/oder Handbreite einer Hand eines Fahrradfahrers für eine durch eine Steuerung bestimmte Auswahl von zumindest einem Fahrradgriff gemäß einer nicht erfindungsgemäßen Ausführungsform,
- Figur 7: eine schematische Darstellung einer Bestimmung einer Schrittlänge eines Fahrradfahrers für eine durch eine Steuerung bestimmte Auswahl von zumindest einem Fahrradrahmen gemäß einer nicht erfindungsgemäßen Ausführungsform,
- Figur 8: eine schematische Darstellung einer Bestimmung eines Kopfumfangs eines Fahrradfahrers für eine durch eine Steuerung bestimmte Auswahl von zumindest einem Fahrradhelm gemäß einer nicht erfindungsgemäßen Ausführungsform,
- Figur 9: eine schematische Darstellung eines Verfahrens zum Auswählen zumindest eines Fahrradsattels für einen Fahrradfahrer gemäß einer Ausführungsform, zeigt, und
- Figur 10: eine schematische Darstellung einer Kommunikation zwischen einem Mobilgerät und einer externen Datenverarbeitungseinrichtung zum Ausführen des in Figur 9 dargestellten Verfahrens zeigt.

### Detaillierte Beschreibung der Figuren

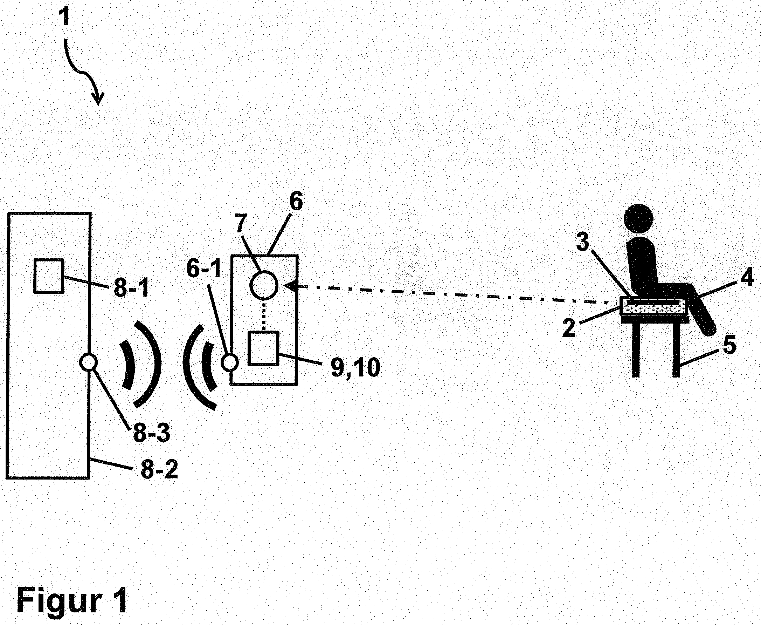

Figur 1 zeigt ein System zum Auswählen zumindest eines Fahrradsattels für einen Fahrradfahrer gemäß einer Ausführungsform in einer schematischen Darstellung.

Das System 1 umfasst eine lediglich schematisch dargestellte Erfassungsvorrichtung 2, welche ausgebildet ist, einen lediglich schematisch dargestellten Gesäßabdruck 3 eines Fahrradfahrers 4 zu erfassen.

Der in Figur 1 lediglich schematisch dargestellte Fahrradfahrer 4 sitzt auf einer schematisch dargestellten Sitzgelegenheit 5, z.B. auf einem Hocker, wobei die Erfassungsvorrichtung 2 insbesondere zwischen der Sitzgelegenheit 5 und dem Gesäß des Fahrradfahrers 4 angeordnet ist, um den Gesäßabdruck 3 zu erfassen.

Auch wenn dies in der Figur 1 nicht dargestellt ist, umfasst der Gesäßabdruck 3 jeweils einen Druckbereich der beiden Sitzknochen des auf der Erfassungsvorrichtung 2 sitzenden Fahrradfahrers 4.

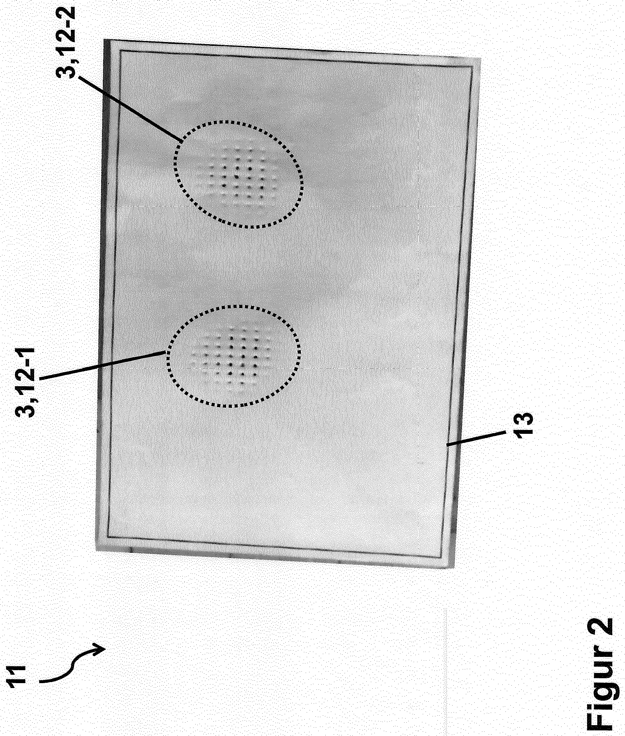

Die Erfassungsvorrichtung 2 kann insbesondere eine in Figur 1 nicht dargestellte Grundplatte mit Noppen umfassen, auf welchen ein Blatt Papier angeordnet ist, wobei der Fahrradfahrer 4 während des Erfassens des Gesäßabdrucks 3 mit seinem Gesäß auf dem Papier sitzt, und wobei während des Erfassens des Gesäßabdrucks 3 die Noppen durch das Papier hindurchgedrückt werden, um die Druckbereiche der beiden Sitzknochen des Fahrradfahrers 4 auf dem Papier zu erhalten. Für weitere Details zum entsprechenden Gesäßabdruck 3 wird auf die nachfolgende Figur 2 verwiesen.

Die Erfassungsvorrichtung 2 gemäß der vorliegenden Anmeldung ist jedoch nicht auf eine bestimmte Vorrichtung beschränkt, sondern kann beliebige Vorrichtungen umfassen, welche zum Erfassen eines Gesäßabdrucks 3 eines Fahrradfahrers 4 ausgebildet sind. Insbesondere umfasst die Erfassungsvorrichtung 2 ein lichttransparentes mit Gel gefülltes Sitzkissen, wobei der Fahrradfahrer 4 während des Erfassens des Gesäßabdrucks 3 mit seinem Gesäß auf dem Sitzkissen sitzt, und wobei während des Erfassens des Gesäßabdrucks 3 sich die Verteilung des Gels innerhalb des Sitzkissens verändert, um eine visuelle Darstellung der Druckbereiche der beiden Sitzknochen des Fahrradfahrers 4 auf dem Sitzkissen zu erhalten.

Das System 1 umfasst ferner ein Mobilgerät 6 mit einer Kamera 7, sowie eine in Figur 1 lediglich schematisch dargestellte Steuerung 8-1 einer externen Datenverarbeitungseinrichtung 8-2. Hierbei weist das Mobilgerät 6 insbesondere eine erste Kommunikationseinrichtung 6-1 auf, welche mit einer zweiten Kommunikationseinrichtung 8-3 der externen Datenverarbeitungseinrichtung 8-2 kommuniziert, insbesondere über eine drahtlose Kommunikationsverbindung. Die Datenverarbeitungseinrichtung 8-2 ist insbesondere als ein externer Server ausgebildet. Das Mobilgerät 6 ist insbesondere als ein Smart-Phone, als ein Smart-Pad, als ein Notebook oder als eine Smart-Watch ausgebildet.

Auch wenn dies in Figur 1 lediglich schematisch dargestellt ist, ist die Kamera 7 des Mobilgeräts 6 ausgebildet, ein Foto des erfassten Gesäßabdrucks 3 des Fahrradfahrers 4 aufzunehmen.

Offensichtlich muss der durch die Erfassungsvorrichtung 2 erfasste Gesäßabdruck 3 im Fokus der Kamera 7 des Mobilgeräts 6 positioniert werden, damit die Kamera 7 das Foto des erfassten Gesäßabdrucks 3 aufnehmen kann. Auch wenn dies in der Figur 1 nicht dargestellt ist, muss hierfür entweder die Erfassungsvorrichtung 2 zwischen dem Gesäß des Fahrradfahrers 4 und der Sitzgelegenheit 5 entfernt werden oder der Fahrradfahrer 4 verlässt einfach nur die Erfassungsvorrichtung, um den Gesäßabdruck 3 anschließend im Fokus der Kamera 7 zu positionieren und das Foto des Gesäßabdrucks 3 aufzunehmen.

Aus darstellungstechnischen Gründen ist der entsprechende Aufnahmevorgang in der Figur 1 nur schematisch durch einen Pfeil dargestellt.

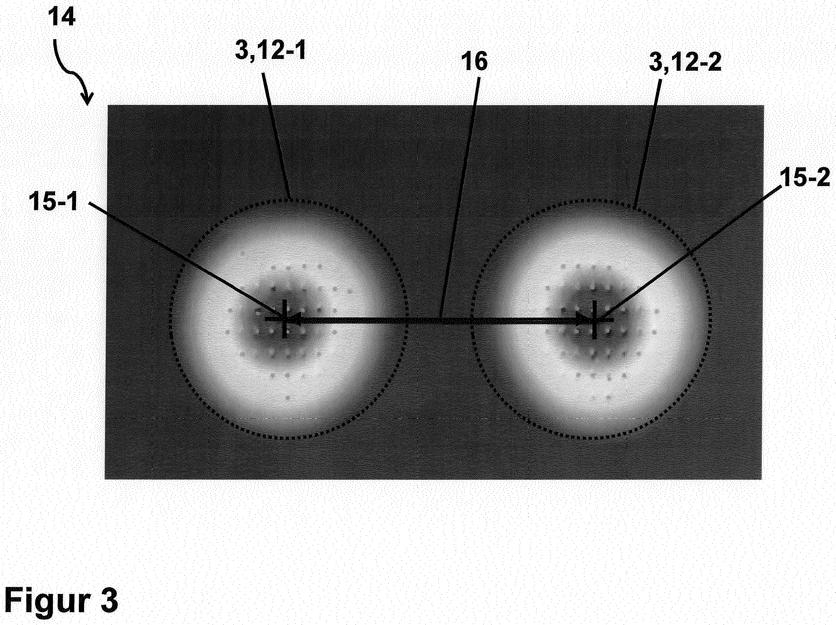

Die mit der Kamera 7 über die Kommunikationsverbindung steuerungstechnisch verbundene Steuerung 8-1 der Datenverarbeitungseinrichtung 8-2, insbesondere in Kombination mit einem auf dem Mobilgerät 6 ausgeführten Programm bzw. App, ist ausgebildet, auf Basis des aufgenommenen Fotos des Gesäßabdrucks 3 einen Sitzknochenabstand des Fahrradfahrers 4 zu bestimmen. Für weitere Details zur Bestimmung des Sitzknochenabstands des Fahrradfahrers 4 durch die Steuerung 8-1 wird auf die nachfolgende Figur 3 verwiesen.

Die Steuerung 8-1 ist ferner ausgebildet, jeweils eine Satteleignung einer Mehrzahl von Fahrradsatteln einer Vielzahl von in einer Datenbank 8-4 der externen Datenverarbeitungseinrichtung 8-2 hinterlegten Fahrradsätteln in Abhängigkeit des bestimmten Sitzknochenabstands und in Abhängigkeit von zumindest einem Eingabeparameter, welcher durch eine Eingabevorrichtung 9 des Mobilgeräts 6 eingegeben wird, zu bestimmen. Die Eingabevorrichtung 9 umfasst insbesondere einen Touch-Display und/oder einen Touch-Button des Mobilgeräts 6.

Wurden von demselben Fahrradfahrer 4 bereits in der Steuerung 8-1, insbesondere durch ein auf dem Mobilgerät 6 ausgeführten Programm bzw. App, bereits Eingabeparameter hinterlegt, können diese bereits hinterlegten Eingabeparameter verwendet werden, um auf deren Basis die Bestimmung der jeweiligen Satteleignung durch die Steuerung 8-1 durchzuführen.

Der eingegebene zumindest eine Eingabeparameter ist insbesondere ausgewählt aus, Fahrradtyp, Geschlecht des Fahrradfahrers 4, Sitzposition des Fahrradfahrers 4 auf dem Fahrradsattel, Sattelbreite des Fahrradsattels, Fahrhäufigkeit des Fahrradfahrers 4, üblicher Dauer einer Fahrradfahrt des Fahrradfahrers 4, Körpergewicht des Fahrradfahrers 4, und/oder anatomische Besonderheiten des Fahrradfahrers 4.

Die Auswahl des Fahrradtyps kann insbesondere die folgenden Unterauswahlen umfassen, Triathlon, Mountainbike XC, Trekking Cross, City Comfort, Rennrad, Mountainbike All Mountain, Trekking Cross, Gravel. Die Auswahl des Fahrradtyps kann insbesondere die Unterauswahl umfassen, ob ein E-Bike gewünscht ist, im Rahmen einer "Ja" oder "Nein" Beantwortung.

Die Auswahl der Sitzposition des Fahrradfahrers 4 auf dem Fahrradsattel kann insbesondere die folgenden Unterauswahlen umfassen: Triathlon, moderat, aufrecht, sportlich, leicht gebeugt.

Insbesondere wird die Sattelbreite des Fahrradsattels durch die Steuerung 8-1 auf Basis des bestimmten Sitzknochenabstands bestimmt, insbesondere dazu zusätzlich auf Basis der ausgewählten Sitzposition des Fahrradfahrers 4.

Die Auswahl der Fahrhäufigkeit kann insbesondere die folgenden Unterauswahlen umfassen: mehrmals die Woche, einmal die Woche, alle zwei Wochen, alle vier Wochen.

Die Auswahl der üblichen Dauer einer Fahrradfahrt des Fahrradfahrers 4 kann insbesondere die folgenden Unterauswahlen umfassen: weniger als 45 Minuten, 45 Minuten bis zwei Stunden, mehr als zwei Stunden.

Die Auswahl des Körpergewichts des Fahrradfahrers 4 kann insbesondere die folgenden Unterauswahlen umfassen: bis 60 kg, bis 80 kg, bis 100 kg, bis 120 kg, über 120 kg.

Die Auswahl von anatomischen Besonderheiten des Fahrradfahrers 4 kann insbesondere die folgende Unterauswahl umfassen: Taubheitsgefühle, Beckenfehlstellung, Steißbein, Schambein, Sitzknochen, Hitze/Schweiß, unterer Rücken.

Insbesondere bestimmt die Steuerung 8-1 die jeweilige Satteleignung wie folgt:
Dem durch die Steuerung 8-1 bestimmten Sitzknochenabstand und dem zumindest einen Eingabeparameter wird durch die Steuerung 8-1 jeweils ein numerischer Wert zugeordnet.

Anschließend werden die jeweils zugeordneten numerischen Werte durch die Steuerung 8-1 gewichtet, um gewichtete numerische Werte zu erhalten. Die Gewichtung umfasst hierbei insbesondere jeweils einen Gewichtungsfaktor, welcher mit dem jeweiligen numerischen Wert multipliziert wird, um den jeweiligen gewichteten numerischen Wert zu erhalten.

Insbesondere beträgt der Gewichtungsfaktor bei der Gewichtung des numerischen Wertes, welcher dem Fahrradtyp des Fahrradsattels zugeordnet ist, zwei. Insbesondere beträgt der Gewichtungsfaktor bei der Gewichtung des numerischen Wertes, welcher der Fahrhäufigkeit des Fahrradfahrers 4, der üblichen Dauer einer Fahrradfahrt des Fahrradfahrers 4, dem Körpergewicht des Fahrradfahrers 4, und/oder anatomische Besonderheiten des Fahrradfahrers 4 zugeordnet ist, eins.

Anschließend wird durch die Steuerung 8-1 ein Mittelwert aus der Summe der gewichteten numerischen Werte gebildet, um die jeweilige Satteleignung zu erhalten, wobei die jeweilige Satteleignung insbesondere durch einen Prozentwert ausgegeben wird.

Anschließend bestimmt die Steuerung 8-1 eine priorisierte Liste der Mehrzahl von Fahrradsätteln, wobei in der priorisierten Liste die Priorität der jeweiligen Fahrradsättel mit zunehmender Satteleignung steigt.

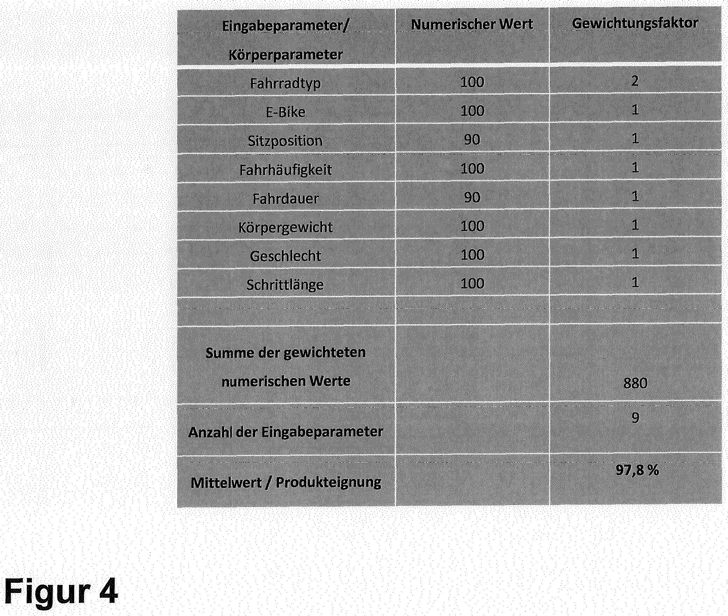

Für weitere Details in Bezug auf die Bestimmung einer analog bestimmten Rahmeignung für einen Fahrradrahmen wird auf die nachfolgende Figur 4 verwiesen.

Das Mobilgerät 6, insbesondere eine in Figur 1 lediglich schematisch dargestellte Ausgabevorrichtung 10 des Mobilgeräts 6, wie z.B. das Display, ist ausgebildet, eine Auswahl von zumindest einem Fahrradsattel der priorisierten Liste der Mehrzahl von Fahrradsätteln für den Fahrradfahrer 4 auszugeben. Hierbei legt die Steuerung 8-1 die Auswahl des zumindest einen Fahrradsattels derart fest, dass die Auswahl den zumindest einen Fahrradsattel der priorisierten Liste der Mehrzahl von Fahrradsatteln umfasst, dessen jeweilige bestimmte Satteleignung die geringste Differenz oder gar keine Differenz zu einer maximalen Satteleignung, wie z.B. 100 %, aufweist.

Das in der Figur 1 lediglich schematisch dargestellte Touch-Display ist somit als eine Eingabevorrichtung 9 und als eine Ausgabevorrichtung 10 ausgebildet.

Hierbei umfassen die durch die Steuerung 8-1 bestimmten jeweiligen Satteleignungen insbesondere numerische Werte bzw. Prozentwerte. Somit kann durch die Steuerung 8-1 bestimmt werden, welche numerischen Werte, bzw. Prozentwerte der jeweiligen Satteleignungen der jeweiligen Fahrradsätteln der maximalen Satteleignung, insbesondere 100 %, am nächsten kommen, um die Auswahl von zumindest einem Fahrradsattel für den Fahrradfahrer 4 auszugeben.

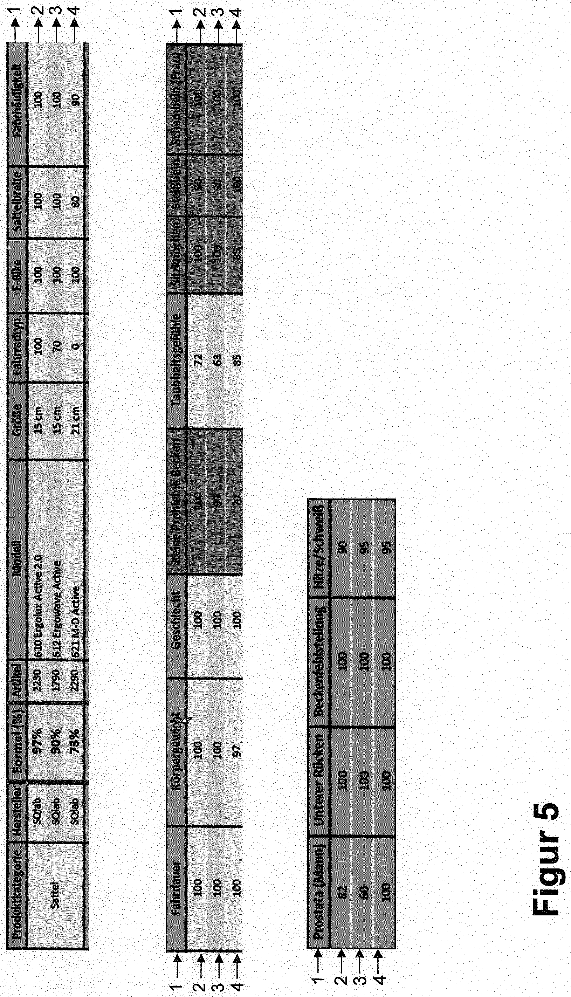

Für eine entsprechende Auswahl von zumindest einem Fahrradsattel wird auf die Figur 5 verwiesen.

Die durch das Mobilgerät 6 für den Fahrradfahrer 4 ausgegebene Auswahl von zumindest einem Fahrradsattel stellt dem Fahrradfahrer 4 somit eine für ihn individualisierte Ausgabe von Fahrradsätteln zur Verfügung, welche für den Fahrradfahrer 4 besonders geeignet sind. Die individualisierte Auswahl, ist insbesondere an die Gesäßform des Fahrradfahrers 4, und/oder an weitere Körperparameter des Fahrradfahrers 4 und/oder an die Fahrgewohnheiten des Fahrradfahrers 4 individuell angepasst.

Figur 2 zeigt einen durch eine Erfassungsvorrichtung erfassten Gesäßabdruck eines Fahrradfahrers.

Der in Figur 2 dargestellte Gesäßabdruck 3 wird durch eine Erfassungsvorrichtung 2 erhalten, welche eine in Figur 2 nicht dargestellte Grundplatte mit Noppen umfasst, auf welcher das in Figur 2 dargestellte Blatt Papier 11 angeordnet ist. Während des Erfassens des Gesäßabdrucks 3 saß der Fahrradfahrer 4 mit seinem Gesäß auf dem Papier 11, so dass die Noppen durch das Papier 11 hindurchgedrückt wurden, um die jeweiligen Druckbereiche 12-1, 12-2 der beiden Sitzknochen des Fahrradfahrers 4 auf dem Papier 11 zu erhalten.

Beispielsweise resultiert der erste Druckbereich 12-1 des Gesäßabdrucks 3 aus dem linken Sitzknochen des Fahrradfahrers 4 und resultiert der zweite Druckbereich 12-2 des Gesäßabdrucks 3 aus dem rechten Sitzknochen des Fahrradfahrers 4.

Auf dem Blatt Papier 11 ist insbesondere ein umlaufender Rahmen 13 angeordnet. Das durch die Steuerung 8-1 durchgeführte Bestimmen eines Sitzknochenabstands umfasst hierbei das Erkennen und Rektifizieren des aufgenommenen Fotos des Gesäßabdrucks 3 auf Basis des umlaufenden Rahmens 13 durch die Steuerung 8-1.

Für weitere Details zum Bestimmen des Sitzknochenabstands durch die Steuerung 8-1 wird auf die nachfolgende Figur 3 verwiesen.

Figur 3 zeigt eine Bestimmung eines Sitzknochenabstands durch eine Steuerung gemäß einer Ausführungsform.

In der Figur 3 ist ein durch eine Kamera 7 des Mobilgeräts 6 aufgenommenes Foto 14 eines Gesäßabdrucks 3 des Fahrradfahrers 4 gezeigt, wobei der Gesäßabdruck 3 einen ersten Druckbereich 12-1 und einen zweiten Druckbereich 12-2 umfasst.

Die Steuerung 8-1, insbesondere im Zusammenspiel mit einem auf dem Mobilgerät 6 ausgeführten Programm bzw. App, bestimmt einen ersten Mittelpunkt 15-1 des in dem aufgenommenen Foto 14 des erfassten Gesäßabdrucks 3 visuell dargestellten ersten Druckbereichs 12-1 eines ersten Sitzknochens des Fahrradfahrers 4.

Die Steuerung 8-1, insbesondere im Zusammenspiel mit einem auf dem Mobilgerät 6 ausgeführten Programm bzw. App, bestimmt einen zweiten Mittelpunkt 15-2 des in dem aufgenommenen Foto 14 des erfassten Gesäßabdrucks 3 visuell dargestellten zweiten Druckbereichs 12-2 eines zweiten Sitzknochens des Fahrradfahrers 4.

Anschließend bestimmt die Steuerung 8-1 einen Abstand 16 zwischen dem ersten und zweiten Mittelpunkt 15-1, 15-2, um den Sitzknochenabstand 16 des Fahrradfahrers 4 zu bestimmen. Der Abstand 16 zwischen dem ersten und zweiten Mittelpunkt 15-1, 15-2 entspricht hierbei dem Sitzknochenabstand 16 des Fahrradfahrers 4.

Figur 4 zeigt eine schematische Darstellung einer durch eine Steuerung bestimmten Produkteignung, insbesondere Rahmeneignung, eines Fahrradprodukts, insbesondere Fahrradrahmens, gemäß einer nicht erfindungsgemäßen Ausführungsform.

In der Figur 4 ist eine beispielhafte Berechnung einer Produkteignung eines Fahrradprodukts, insbesondere eine beispielhafte Berechnung einer Rahmeneignung einer Fahrradrahmens gezeigt. Eine entsprechende Berechnung ist jedoch analog auf andere Fahrradprodukte, insbesondere Fahrradsättel, Fahrradgriffe, und/oder Fahrradhelme anzuwenden.

In der Figur 4 sind eine Vielzahl von Eingabeparametern, insbesondere Fahrradtyp, E-Bike-Auswahl, Sitzposition, Fahrhäufigkeit, Fahrdauer, Körpergewicht, und Geschlecht, sowie die Schrittlänge als bestimmter Körperparameter des Fahrradfahrers 4 dargestellt.

Wie aus der Figur 4 hervorgeht, ordnet die Steuerung 8-1 jedem Eingabeparameter und dem Körperparameter jeweils einen numerischen Wert zu, wobei in dem vorliegenden Ausführungsbeispiel der numerische Wert in einem Bereich von 0 bis 100 gewählt ist, wobei insbesondere der unterste Wert 0 einer besonders negativen Zuordnung entspricht, und wobei insbesondere der oberste Wert 100 einer besonders positiven Zuordnung entspricht. Die numerischen Werte der Eingabeparameter bzw. des als Schrittlänge bestimmten Körperparameters sind in der zweiten Spalte der Tabelle der Figur 4 dargestellt.

Die Steuerung 8-1 gewichtet die jeweils zugeordneten numerischen Werte mit einem Gewichtungsfaktor, um gewichtete numerische Werte zu erhalten. Es wird betont, dass beim Gewichten der numerischen Werte jeweils ein Gewichtungsfaktor mit dem jeweiligen numerischen Wert multipliziert wird, um den jeweiligen gewichteten numerischen Wert zu erhalten. In dem vorliegenden Ausführungsbeispiel beträgt der Gewichtungsfaktor für den Fahrradtyp zwei und beträgt der Gewichtungsfaktor für die weiteren Eingabeparameter und die Schrittlänge eins. Die Gewichtungsfaktoren sind in der dritten Spalte der Tabelle der Figur 4 dargestellt.

Die Steuerung 8-1 addiert hierbei die gewichteten numerischen Werte und teilt die entsprechende Summe der gewichteten numerischen Werte durch die Anzahl der numerischen Werte (insbesondere multipliziert mit dem jeweiligen Gewichtungsfaktor), um als resultierenden Mittelwert die entsprechende Produkteignung, insbesondere Rahmeneignung zu erhalten, wie folgt: Produkteignung (%) = Σ (numerischer Wert x Gewichtungsfaktor) / Σ (Anzahl der numerischen Wert x Gewichtungsfaktor)

In der vorliegenden Figur 4 beträgt die Summe der gewichteten numerischen Werte 880 und beträgt die Anzahl der numerischen Werte multipliziert mit dem jeweiligen Gewichtungsfaktor 9, da hierbei die Gewichtung des Fahrradtyps mit dem Gewichtungsfaktor 2 bei der Anzahl der numerischen Werte doppelt gezählt wird.

Somit ergibt sich aus der Division des Wertes 880 durch den Wert 9 als Mittelwert, bzw. als Produkteignung, bzw. Rahmeneignung ein Wert von 97,8 %.

Figur 5 zeigt eine schematische Darstellung einer durch eine Steuerung bestimmten Auswahl von zumindest einem Fahrradprodukt, insbesondere Fahrradsattel gemäß einer Ausführungsform.

Figur 5 zeigte eine durch ein Mobilgerät angezeigte Auswahl einer priorisierten Liste einer Mehrzahl von Fahrradprodukten, insbesondere Fahrradsätteln, wobei in der priorisierten Liste die Priorität der jeweiligen Fahrradprodukte, insbesondere Fahrradsättel, mit zunehmender Produkteignung, insbesondere Satteleignung, steigt.

Wie aus der Figur 5 ersichtlich ist, wurde eine Vielzahl von Eingabeparametern des Fahrradfahrers 4 durch das Mobilgerät 6 eingegeben, so dass die in Figur 5 dargestellte priorisierte Liste eine Vielzahl von Spalten aufweist, in welchen für den jeweiligen Fahrradsattel und für die jeweiligen Eingabeparameter jeweils ein gewichteter numerischer Wert zugeordnet ist. Die gewichteten numerischen Werte gehen in die Gesamtberechnung der jeweiligen Produkteignung ein, wie in der Figur 5 dargestellt ist. In der Gesamtberechnung ergibt sich dann unter Berücksichtigung aller numerischen Werte in der priorisierte Liste ein Ranking der jeweiligen Satteleignung (in der Figur 5 als "Formel" bezeichnet) von 97%, 90 % bzw. 73 % für die drei in Figur 5 gezeigten Fahrradsättel.

Zur besseren Darstellbarkeit wurde in der Figur 5 die Vielzahl von Spalten in unterschiedlichen graphischen Darstellungen übereinander angeordnet, wobei die sich in den graphischen Darstellungen jeweils fortsetzenden Zeilen durch Pfeilmarkierungen und mit den Zahlen 1, 2, 3 und 4 zugeordnet werden können.

Somit weist der in der Figur 5 dargestellte Fahrradsattel SQlab 621 M-D Active mit 73% die niedrigste Satteleignung der Mehrzahl von Fahrradsätteln auf, weist der in der Figur 5 dargestellte Fahrradsattel SQlab 610 Ergolux Active 2.0 mit 97% die höchste Satteleignung der Mehrzahl von Fahrradsätteln auf und weist der in der Figur 5 dargestellte Fahrradsattel SQlab 612 Ergowave Active mit 90% die zweithöchste Satteleignung der Mehrzahl von Fahrradsätteln auf.

Die in der Figur 5 gezeigten Fahrradprodukte, insbesondere Fahrradsättel weisen somit im Vergleich zu weiteren Fahrradsätteln der priorisierten Liste eine besonders geringe Differenz zu einer maximalen Produkteignung, insbesondere Satteleignung auf, welche insbesondere 100% umfasst.

Somit umfasst die in Figur 5 dargestellte Auswahl von Fahrradsätteln insbesondere die Fahrradsättel deren jeweilige Differenz zwischen der jeweiligen Satteleignung und der maximalen Satteleignung geringer als einen Schwellenwert ist, wobei der Schwellenwert im vorliegenden Fall insbesondere weniger als 30% beträgt, so dass in diesem Fall insbesondere die Produkteignung der in der Figur 5 dargestellten Fahrradsättel insbesondere mehr als 70% beträgt.

Somit kann eine besonders vorteilhafte in Bezug auf den Fahrradfahrer 4 individualisierte Ausgabe von Fahrradsätteln sichergestellt werden.

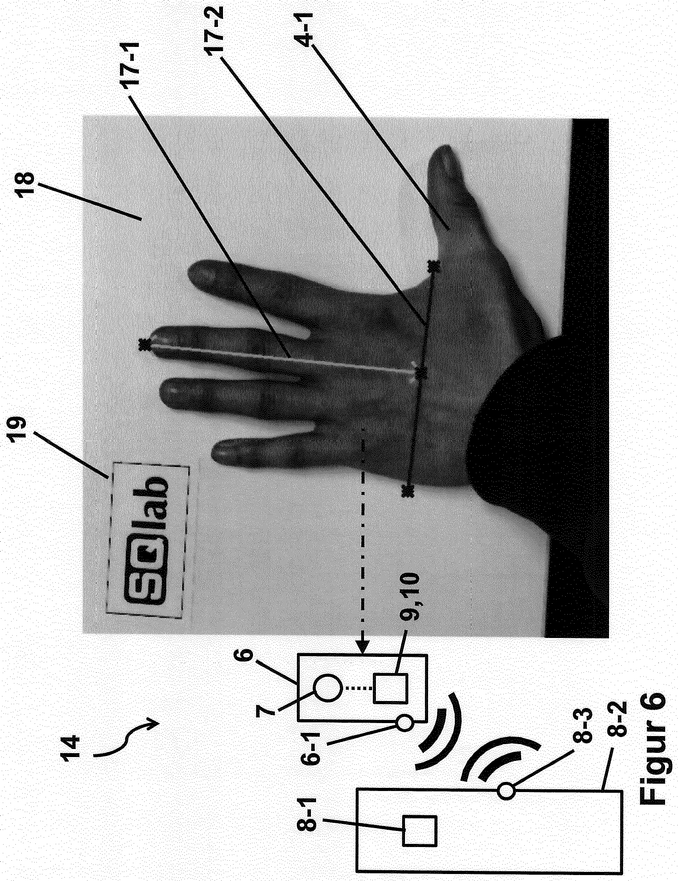

Figur 6 zeigt eine schematische Darstellung einer Bestimmung einer Handlänge und/oder Handbreite einer Hand eines Fahrradfahrers für eine durch eine Steuerung 8-1 bestimmte Auswahl von zumindest einem Fahrradgriff gemäß einer nicht erfindungsgemäßen Ausführungsform.

Die in Figur 6 lediglich schematisch dargestellte Steuerung 8-1 ist ferner insbesondere zum Auswählen von zumindest einem Fahrradgriff für den Fahrradfahrer 4 ausgebildet.

Hierzu nimmt die Kamera 7 des Mobilgeräts 6 ein Foto 14 einer Hand 4-1 des Fahrradfahrers 4 auf. Hierbei legt der Fahrradfahrer 4, wie in Figur 6 schematisch dargestellt ist, seine Hand 4-1 insbesondere auf einen Referenzhintergrund 18, welche insbesondere eine helle Oberfläche umfasst. Wie aus der Figur 6 hervorgeht, ist auf dem Referenzhintergrund 18 ein Referenzsymbol 19 mit einer definierten Größe angeordnet. Durch dieses angeordnete Referenzsymbol 19 mit einer definierten Größe kann die Steuerung 8-1 die bestimmte Handlänge 17-1 und/oder die bestimmte Handbreite 17-2 zu der definierten Größe des Referenzsymbols 19 in Bezug setzen, um dadurch die Abmessungen der Handlänge 17-1 und/oder Handbreite 17-2 präzise zu bestimmen.

Wie in der Figur 6 lediglich schematisch dargestellt ist, bestimmt die Steuerung 8-1 eine Handlänge 17-1 und/oder eine Handbreite 17-2 der Hand 4-1 des Fahrradfahrers 4 auf Basis des Fotos 14 der Hand 4-1. Insbesondere bestimmt die Steuerung 8-1 die Handlänge 17-1 und/oder die Handbreite 17-2 der Hand 4-1 des Fahrradfahrers 4 auf Basis des Fotos 14 der Hand 4-1 und auf Basis der definierten Größe des Referenzsymbols 19.

Das Bestimmen der Handlänge 17-1 und/oder der Handbreite 17-2 der Hand 4-1 des Fahrradfahrers 4 umfasst insbesondere die folgenden Schritte, welche insbesondere unter Verwendung des Programms "Mediapipe" durchgeführt werden (https://www.mediapipe.dev/).

In einem ersten Schritt wird durch die Steuerung 8-1 der Verlauf des Skeletts der Hand 4-1 des Fahrradfahrers 4 erkannt.

In einem zweiten Schritt wird durch die Steuerung 8-1 eine erste Gerade bestimmt, die durch den Mittelfinger verläuft, wobei die erste Gerade an der Spitze des Mittelfingers beginnt und auf der Höhe des Daumens endet. Dabei wird der Beginn des Daumens senkrecht auf die erste Gerade projiziert. Um die erste Gerade zu berechnen, werden die Punkte des Mittelfingers linear interpoliert.

Um auf Basis der ersten Gerade die Spitze des Mittelfingers und den Daumen zu finden, wird die Hand 4-1 zuerst segmentiert. Hierbei wird auf der Höhe der Fingerknöchel eine in Figur 6 nicht dargestellte zweite Gerade konstruiert, die senkrecht zur ersten Gerade verläuft.

Entlang dieser zweiten Gerade wird nach den zwei Rändern der Segmentierung gesucht. Wenn die Schnittpunkte mit dem Rand der Hand 4-1 gefunden sind wird deren Abstand bestimmt. Danach wird die zweite Gerade iterativ zum Handgelenk bewegt. Bei jeder Iteration wird die Distanz der zweiten Gerade geprüft.

Wenn eine geprüfte Distanz der zweiten Gerade mehr als 10% größer ist als die vorherig geprüfte Distanz der zweiten Gerade ist, dann wurde der Daumen gefunden. Die vorherig geprüfte Distanz der zweiten Gerade entspricht der in Figur 6 dargestellten Handbreite 17-2 der Hand 4-1.

Um die Fingerspitze des Mittelfingers zu finden, wird entlang der ersten Gerade nach einem Schnittpunkt mit dem Rand der Hand 4-1 gesucht, um die Handlänge 17-1 zu erhalten.

Die Steuerung 8-1 bestimmt eine jeweilige Griffeignung einer Mehrzahl von in einer Datenbank 8-3 einer externen Datenverarbeitungseinrichtung 8-2 hinterlegten Fahrradgriffen in Abhängigkeit von der bestimmen Handlänge 17-1 und/oder der bestimmten Handbreite 17-2, sowie in Abhängigkeit von zumindest einem in der Steuerung 8-1 hinterlegten Eingabeparameter.

Das Bestimmen der jeweiligen Griffeignung durch die Steuerung 8-1 erfolgt analog zum Bestimmen der jeweiligen Satteleignung, wobei jedoch gegebenenfalls unterschiedliche Parameter bzw. Gewichtungen herangezogen werden.

Der zumindest eine eingegebene Eingabeparameter ist insbesondere ausgewählt aus, Fahrradtyp, Fahrhäufigkeit des Fahrradfahrers 4, üblicher Dauer einer Fahrradfahrt des Fahrradfahrers 4, Sitzposition des Fahrradfahrers 4 auf dem Fahrrad, Griffweite des Fahrradfahrers 4, Körpergröße des Fahrradfahrers 4, und/oder anatomische Besonderheiten des Fahrradfahrers 4.

Die Auswahl der Griffweite kann insbesondere die folgende Unterauswahl umfassen: Small (S), Medium (M), Large (L), Extra Large (XL).

Die Auswahl von anatomischen Besonderheiten des Fahrradfahrers 4 kann insbesondere die folgende Unterauswahl umfassen: tauber Daumen, Zeige- und Mittelfinger, tauber kleiner Finger und Ringfinger, Handgelenk, oberer Rücken.

Insbesondere umfasst das Bestimmen der jeweiligen Griffeignung die folgenden Schritte:
Die Steuerung 8-1 ordnet jeweils einen numerischen Wert zu der bestimmten Handbreite 17-2 und/oder zu der bestimmten Handlänge 17-1 und zu dem zumindest einen Eingabeparameter zu.

Die Steuerung 8-1 gewichtet anschließend die jeweils zugeordneten numerischen Werte, um gewichtete numerische Werte zu erhalten.

Die Steuerung 8-1 bildet anschließend einen Mittelwert aus der Summe der gewichteten numerischen Werte, um die jeweilige Griffeignung zu erhalten.

Insbesondere umfasst der zumindest eine Eingabeparameter den Fahrradtyp, wobei insbesondere der Gewichtungsfaktor bei der Gewichtung des numerischen Wertes, welcher dem Fahrradtyp des Fahrradsattels zugeordnet ist, zwei beträgt.

Insbesondere umfasst der zumindest eine Eingabeparameter ferner eine Fahrhäufigkeit des Fahrradfahrers 4, eine übliche Dauer einer Fahrradfahrt des Fahrradfahrers 4, ein Körpergewicht des Fahrradfahrers 4, eine Sitzposition des Fahrradfahrers 4 auf dem Fahrrad, eine Griffweite des Fahrradfahrers 4, eine Körpergröße des Fahrradfahrers 4, und/oder anatomische Besonderheiten des Fahrradfahrers 4, wie z.B. keine Probleme, tauber Daumen, Zeige-, und Mittelfinger, tauber kleiner Finger und Ringfinger, Handgelenk, und/oder oberer Rücken, wobei insbesondere der Gewichtungsfaktor bei der Gewichtung des jeweiligen numerischen Wertes, welcher dem jeweiligen Eingabeparameter zugeordnet ist, eins beträgt.

Die Steuerung 8-1 gibt anschließend eine Auswahl von zumindest einem Fahrradgriff für den Fahrradfahrer 4 aus, deren jeweilige Griffeignung zu einer maximalen Griffeignung, insbesondere 100%, die geringste Differenz oder gar keine Differenz aufweist.

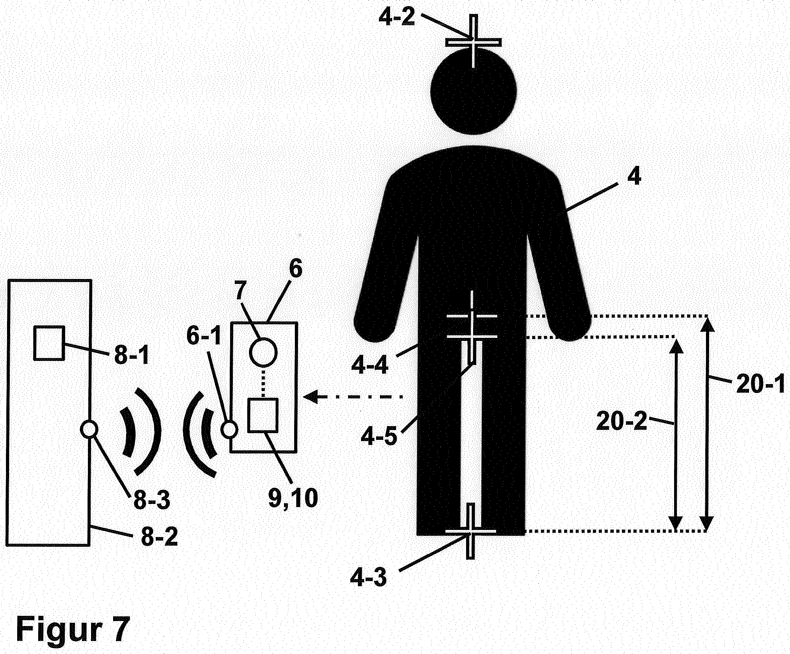

Figur 7 zeigt eine schematische Darstellung einer Bestimmung einer Schrittlänge eines Fahrradfahrers für eine durch eine Steuerung bestimmte Auswahl von zumindest einem Fahrradrahmen gemäß einer nicht erfindungsgemäßen Ausführungsform.

Die in Figur 1 und Figur 7 lediglich schematisch dargestellte Steuerung 8-1 ist ferner insbesondere zum Auswählen von zumindest einem Fahrradrahmen und/oder eines Fahrrades für den Fahrradfahrer 4 ausgebildet.

Hierzu nimmt die Kamera 7 des Mobilgeräts 6 ein Foto 14 des gesamten Fahrradfahrers 4 auf, und die Steuerung 8-1 bestimmt eine Schrittlänge des Fahrradfahrers 4 auf Basis des Fotos 14 des gesamten Fahrradfahrers 4, insbesondere unter Verwendung des Programms "Mediapipe" (https://www.mediapipe.dev/).

Beim Aufnehmen des Fotos 14 des Fahrfahrradfahrers 4 ist es insbesondere von Vorteil, wenn der Fahrradfahrer 4 von vorne fotografiert wird, wenn der Fahrradfahrer 4 einen Großteil des Fotos 14 einnimmt, wenn alle Körperteile des Fahrradfahrers 4 sichtbar sind, wenn keine weiteren Personen auf dem Foto 14 abgebildet sind, wenn die Füße des Fahrradfahrers 4 in einer V-Ausrichtung stehen und die Fersen gut erkennbar sind, und/oder wenn der Aufnahmewinkel ca. 90° beträgt.

Wie in der Figur 7 schematisch gezeigt ist, bestimmt die Steuerung 8-1 auf dem aufgenommenen Foto 14 einen obersten Punkt 4-2, insbesondere an dem Skalp, des Fahrradfahrers 4, und bestimmt die Steuerung 8-1 auf dem aufgenommenen Foto 14 einen untersten Punkt 4-3, insbesondere an dem Mittelpunkt zwischen den beiden Fersen, des Fahrradfahrers 4.

Anschließend bestimmt die Steuerung 8-1 auf dem aufgenommenen Foto 14 einen mittleren Punkt 4-4, insbesondere an der Hüfte, des Fahrradfahrers 4.

Anschließend bestimmt die Steuerung 8-1 auf dem aufgenommenen Foto 14 den Abstand 20-1 zwischen dem untersten Punkt 4-3 und dem mittleren Punkt 4-4. Die Steuerung 8-1 reduziert anschließend den Abstand 20-1 um ca. 5%, um die Schrittlänge 20-2 des Fahrradfahrers 4 zwischen dem untersten Punkt 4-3 und einem Schrittpunkt 4-5 des Fahrradfahrers 4 zu bestimmen.

Die Steuerung 8-1 bestimmt die jeweilige Rahmeneignung in Abhängigkeit von der bestimmten Schrittlänge 20-2, sowie in Abhängigkeit von zumindest einem eingegebenen Eingabeparameter.

Das Bestimmen der jeweiligen Rahmeneignung durch die Steuerung 8-1 erfolgt analog zum Bestimmen der jeweiligen Satteleignung und/oder der jeweiligen Griffeignung, wobei jedoch gegebenenfalls unterschiedliche Parameter bzw. Gewichtungen herangezogen werden.

Der zumindest eine eingegebene Eingabeparameter ist insbesondere ausgewählt aus, Geschlecht des Fahrradfahrers 4, gewünschter Fahrradtyp des Fahrradfahrers 4, Fahrhäufigkeit des Fahrradfahrers 4, üblicher Dauer einer Fahrradfahrt des Fahrradfahrers 4, Sitzposition des Fahrradfahrers 4 auf dem Fahrradsattel, Körpergewicht des Fahrradfahrers 4, Körpergröße des Fahrradfahrers 4, Schuhgröße des Fahrradfahrers 4, Fußtyp des Fahrradfahrers 4, Fußstellung des Fahrradfahrers 4, Beinstellung des Fahrradfahrers 4, und/oder anatomische Besonderheiten des Fahrradfahrers 4.

Die Auswahl der Beinstellung des Fahrradfahrers 4 kann insbesondere die folgenden Unterauswahlen umfassen: O-Beine, gerade Beine, X-Beine.

Die Auswahl des Fußtyps des Fahrradfahrers 4 kann insbesondere die folgenden Unterauswahlen umfassen: Hohlfuß, Normalfuß, Senkfuß, Plattfuß.

Die Auswahl der Fußstellung des Fahrradfahrers 4 kann insbesondere die folgenden Unterauswahlen umfassen: V-förmig, gerade.

Die Auswahl der Schuhgröße des Fahrradfahrers 4 kann insbesondere in Zentimeter angegeben werden. Sie kann aber auch in jeder anderen gängigen Schuhgröße angegeben werden.

Die Auswahl von anatomischen Besonderheiten des Fahrradfahrers 4 kann insbesondere die folgende Unterauswahl umfassen: Taubheitsgefühle Zehen, Kniebeschwerden.

Insbesondere umfasst das Bestimmen der jeweiligen Rahmeneignung die folgenden Schritte:
Die Steuerung 8-1 ordnet jeweils einen numerischen Wert zu der bestimmten Schrittlänge 20-2 und zu dem zumindest einen Eingabeparameter zu.

Die Steuerung 8-1 gewichtet anschließend die jeweils zugeordneten numerischen Werte, um gewichtete numerische Werte zu erhalten.

Die Steuerung 8-1 bildet anschließend einen Mittelwert aus der Summe der gewichteten numerischen Werte, um die jeweilige Rahmeneignung zu erhalten.

Nach dem Bestimmen der priorisierten Liste durch die Steuerung gibt das Mobilgerät 6 anschließend eine Auswahl von zumindest einem Fahrradrahmen und/oder ein Fahrrad für den Fahrradfahrer 4 aus, wobei die Auswahl den zumindest einen Fahrradrahmen der Mehrzahl von Fahrradrahmen oder das zumindest eine Fahrrad der Mehrzahl von Fahrrädern umfasst, dessen jeweilige bestimmte Rahmeneignung die geringste Differenz oder gar keine Differenz zu einer maximalen Rahmeneignung aufweist.

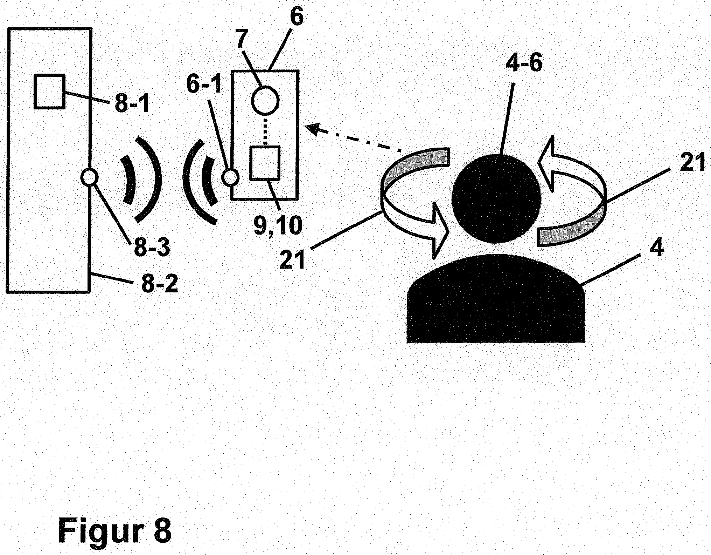

Figur 8 zeigt eine schematische Darstellung einer Bestimmung eines Kopfumfangs eines Fahrradfahrers für eine durch eine Steuerung bestimmte Auswahl von zumindest einem Fahrradhelm gemäß einer nicht erfindungsgemäßen Ausführungsform.

Die in Figur 8 lediglich schematisch dargestellte Steuerung 8-1 ist ferner insbesondere zum Auswählen von zumindest einem Fahrradhelm für den Fahrradfahrer 4 ausgebildet.

Hierzu nimmt die Kamera 7 des Mobilgeräts 6 ein Foto 14 des Kopfes 4-6 des Fahrradfahrers 4 auf, und die Steuerung 8-1, insbesondere in Kombination mit einem auf dem Mobilgerät 6 ausgeführten Programm bzw. App, bestimmt einen Kopfumfang 21 des Fahrradfahrers 4 auf Basis des Fotos 14 des Fahrradfahrers 4, insbesondere unter Verwendung des Programms "Mediapipe" (https://www.mediapipe.dev/).

Insbesondere umfasst die Aufnahme eines Fotos 14 des Fahrradfahrers 4, die Aufnahme eines ersten Fotos 14 von der Vorderseite des Fahrradfahrers 4, und die Aufnahme eines zweiten Fotos 14 von der Lateralseite des Fahrradfahrers 4, wobei die Steuerung 8-1 ausgebildet ist, auf Basis des ersten und zweiten Fotos 14 den Kopfumfang 21 des Fahrradfahrers 4 zu bestimmen.

Die Steuerung 8-1 bestimmt jeweils eine Helmeignung in Abhängigkeit von dem bestimmten Kopfumfang 21, sowie in Abhängigkeit von zumindest einem eingegebenen Eingabeparameter.

Das Bestimmen der jeweiligen Helmeignung des Fahrradfahrers 4 durch die Steuerung 8-1 erfolgt analog zum Bestimmen der jeweiligen Satteleignung, wobei jedoch gegebenenfalls unterschiedliche Parameter, bzw. Gewichtungen herangezogen werden.

Der zumindest eine Eingabeparameter ist insbesondere ausgewählt aus Geschlecht des Fahrradfahrers 4, gewünschter Fahrradtyp des Fahrradfahrers 4, Fahrhäufigkeit des Fahrradfahrers 4, üblicher Dauer einer Fahrradfahrt des Fahrradfahrers 4, Sitzposition des Fahrradfahrers 4 auf dem Fahrradsattel, Körpergewicht des Fahrradfahrers 4, Körpergröße des Fahrradfahrers 4, Kopfumfang 21 des Fahrradfahrers 4, Kopfhöhe des Fahrradfahrers 4, Kopfbreite des Fahrradfahrers 4, und/oder anatomische Besonderheiten des Fahrradfahrers 4.

Insbesondere umfasst das Bestimmen die folgenden Schritte:
Die Steuerung 8-1 ordnet jeweils einen numerischen Wert zu dem bestimmten Kopfumfang 21 und zu dem zumindest einen Eingabeparameter zu.

Die Steuerung 8-1 gewichtet anschließend die jeweils zugeordneten numerischen Werte, um gewichtete numerische Werte zu erhalten.

Die Steuerung 8-1 bildet anschließend einen Mittelwert aus der Summe der gewichteten numerischen Werte, um die jeweilige Helmeignung zu erhalten.

Nach dem die Steuerung 8-1 eine priorisierte Liste der Mehrzahl von Fahrradhelmen bestimmt hat, gibt das Mobilgerät 6 anschließend eine Auswahl von zumindest einem Fahrradhelm für den Fahrradfahrer 4 aus, wobei die Auswahl den zumindest einen Fahrradhelm umfasst, dessen jeweilige Helmeignung zu einer maximalen Helmeignung die geringste Differenz oder gar keine Differenz aufweist.

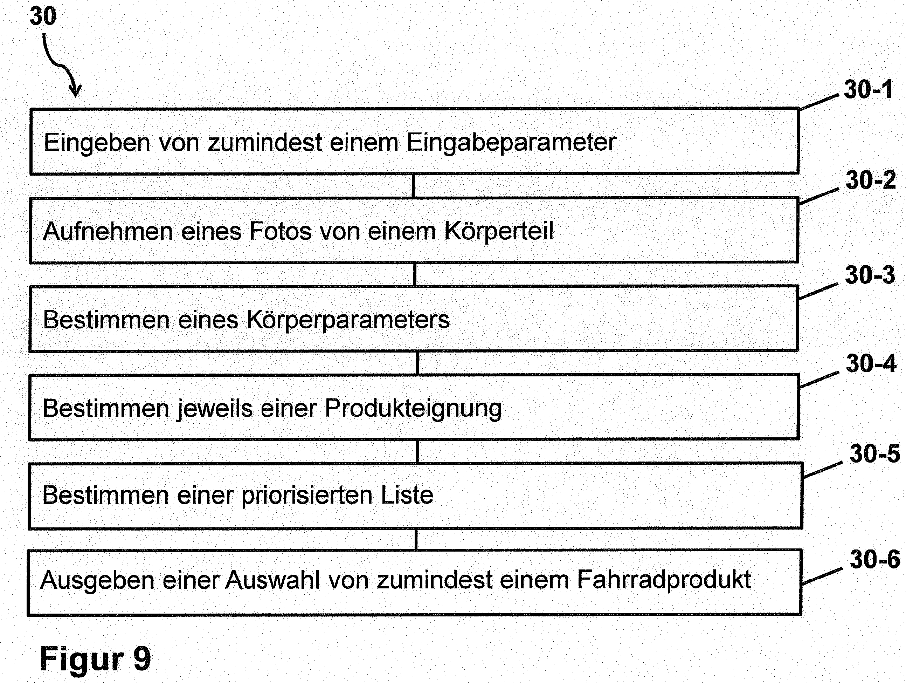

Figur 9 zeigt eine schematische Darstellung eines Verfahrens zum Auswählen zumindest eines Fahrradprodukts für einen Fahrradfahrer 4 gemäß einer Ausführungsform.

Das Verfahren 30 umfasst als ersten Verfahrensschritt das Eingeben 30-1 von zumindest einem Eingabeparameter, insbesondere Geschlecht, Fahrradtyp, Fahrhäufigkeit des Fahrradfahrers 4, Körpergewicht des Fahrradfahrers 4, Sitzposition des Fahrradfahrers 4, anatomische Besonderheiten des Fahrradfahrers 4, Körpergröße des Fahrradfahrers 4, Beinstellung des Fahrradfahrers 4, Fußtyp des Fahrradfahrers 4, Schuhgröße des Fahrradfahrers 4, Fußstellung des Fahrradfahrers 4, und/oder eine übliche Dauer einer Fahrradfahrt des Fahrradfahrers 4, durch eine Eingabevorrichtung 9 eines Mobilgeräts 6.

Das Verfahren 30 umfasst als zweiten Verfahrensschritt das Aufnehmen 30-2 eines Fotos 14 von zumindest einem Körperteil des Fahrradfahrers 4 durch eine Kamera 7 des Mobilgeräts 6.

Das Verfahren 30 umfasst als dritten Verfahrensschritt das Bestimmen 30-3 zumindest eines Körperparameters des Fahrradfahrers 4 auf Basis des aufgenommenen Fotos 14 durch eine Steuerung 8-1.

Das Verfahren 30 umfasst als vierten Verfahrensschritt das Bestimmen 30-4 jeweils einer Produkteignung einer Mehrzahl von Fahrradprodukten der Vielzahl von in der Datenbank hinterlegten Fahrradprodukten in Abhängigkeit des bestimmten Körperparameters und in Abhängigkeit des zumindest einen eingegebenen Eingabeparameters durch die Steuerung 8-1.

Das Verfahren 30 umfasst als fünften Verfahrensschritt das Bestimmen 30-5 einer priorisierten Liste der Mehrzahl von Fahrradprodukten durch die Steuerung 8-1, wobei in der priorisierten Liste die Priorität der jeweiligen Fahrradprodukte mit zunehmender Satteleignung steigt. Das Bestimmen 30-5 einer priorisierten Liste der Mehrzahl von Fahrradprodukten umfasst auch das Erstellen eben dieser Liste.

Das Verfahren 30 umfasst als sechsten Verfahrensschritt das Ausgeben 30-6 einer Auswahl von zumindest einem Fahrradprodukt für den Fahrradfahrer 4 durch das Mobilgerät 6, wobei die Auswahl das zumindest eine Fahrradprodukt der Mehrzahl von Fahrradprodukten umfasst, dessen jeweilige bestimmte Produkteignung die geringste Differenz oder gar keine Differenz zu einer maximalen Produkteignung aufweist.

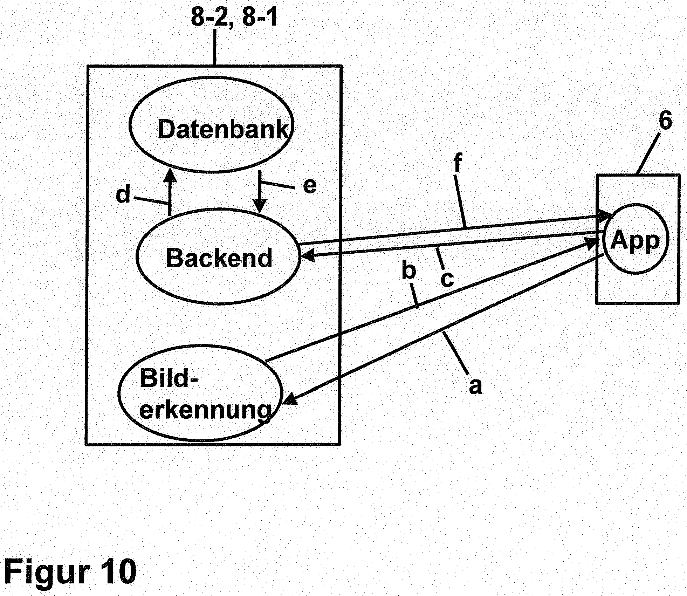

Figur 10 zeigt eine schematische Darstellung einer Kommunikation zwischen einem Mobilgerät und einer externen Datenverarbeitungseinrichtung zum Ausführen des in Figur 9 dargestellten Verfahrens.

In der Figur 10 ist das Mobilgerät 6 mit der darauf ausgeführten App und die externe Datenverarbeitungseinrichtung 8-2 umfassend eine Bilderkennung, ein Backend, und eine Datenbank als einzelne Untermodule der Steuerung 8-1 der Datenverarbeitungseinrichtung 8-2 lediglich schematisch dargestellt.

Nach dem Eingeben 30-1 des zumindest einen Eingabeparameters in das Mobilgerät 6 und dem Aufnehmen 30-2 des Fotos 14 von zumindest einem Körperteil des Fahrradfahrers 4 durch eine Kamera 7 des Mobilgeräts 6, wird das aufgenommene Foto 14 von der App des Mobilgeräts 6 an das Bilderkennungsuntermodul der Steuerung 8-1 der Datenverarbeitungseinrichtung 8-2 übermittelt (dargestellt als Schritt a in Figur 10).

Die Bilderkennung als Untermodul der Steuerung 8-1 bestimmt auf Basis des aufgenommenen Fotos 14 den zumindest einen Körperparameter des Fahrradfahrers 4 und übermittelt den zumindest einen Körperparameter zurück an die App des Mobilgeräts 6 (dargestellt als Schritt b in Figur 10). Somit verfügt die App über den zumindest einen Körperparameter.

Anschließend übermittelt die App des Mobilgeräts 6 den zumindest einen Eingabeparameter und den zumindest einen Körperparameter an das Backenduntermodul der Steuerung 8-1 der Datenverarbeitungseinrichtung 8-2 (dargestellt als Schritt c in Figur 10), wobei das Backend als Schnittstelle zur Weiterübermittlung des zumindest einen Eingabeparameters und des zumindest einen Körperparameters an das Datenbankuntermodul der Steuerung 8-1 dient (dargestellt als Schritt d in Figur 10).

Das Datenbankuntermodul der Steuerung 8-1 steht mit der in Figur 10 nicht dargestellten Datenbank der Datenverarbeitungseinrichtung 8-2 in Verbindung. Das Datenbankmodul bestimmt zuerst in Abhängigkeit von dem zumindest einen Eingabeparameter und dem zumindest einen Körperparameter jeweils eine Produkteignung einer Mehrzahl von Fahrradprodukten einer Vielzahl von in der Datenbank hinterlegten Fahrradprodukten, bestimmt anschließend eine in Abhängigkeit der Produkteignung priorisierte Liste der Mehrzahl von Fahrradprodukten, und übermittelt die priorisierte Liste an das Backend zurück (dargestellt als Schritt e in Figur 10), wobei das Backend die priorisierte Liste weiter an die App des Mobilgeräts 6 zurückübermittelt (dargestellt als Schritt f in Figur 10).

Anschließend führt das Mobilgerät 6 den Verfahrensschritt Ausgeben 30-6 einer Auswahl von zumindest einem Fahrradprodukt für den Fahrradfahrer 4 durch, wobei die Auswahl das zumindest eine Fahrradprodukt umfasst, dessen jeweilige bestimmte Produkteignung eine geringste Differenz oder gar keine Differenz zu einer maximalen Produkteignung aufweist.

### Bezugszeichen

- 1: System zum Auswählen zumindest eines Fahrradsattels
- 2: Erfassungsvorrichtung
- 3: Gesäßabdruck
- 4: Fahrradfahrer
- 4-1: Hand
- 4-2: Oberster Punkt des Fahrradfahrers (Kopf)
- 4-3: Unterster Punkt des Fahrradfahrers (Füße)
- 4-4: Mittlerer Punkt des Fahrradfahrers (Hüfte)
- 4-5: Schrittpunkt des Fahrradfahrers
- 4-6: Kopf des Fahrradfahrers
- 5: Sitzgelegenheit
- 6: Mobilgerät
- 6-1: Erste Kommunikationseinrichtung
- 7: Kamera des Mobilgeräts
- 8-1: Steuerung
- 8-2: Externe Datenverarbeitungseinrichtung
- 8-3: Zweite Kommunikationseinrichtung
- 9: Eingabevorrichtung des Mobilgeräts
- 10: Ausgabevorrichtung des Mobilgeräts
- 11: Papier
- 12-1: Erster Druckbereich
- 12-2: Zweiter Druckbereich
- 13: Umlaufender Rahmen
- 14: Foto
- 15-1: Erster Mittelpunkt
- 15-2: Zweiter Mittelpunkt
- 16: Sitzknochenabstand
- 17-1: Handlänge
- 17-2: Handbreite
- 18: Referenzhintergrund
- 19: Referenzsymbol
- 20-1: Abstand zwischen untersten Punkt 4-3 und mittlerem Punkt 4-4
- 20-2: Schrittlänge
- 21: Kopfumfang
- 30: Verfahren zum Auswählen zumindest eines Fahrradprodukts
- 30-1: Erster Verfahrensschritt: Eingeben von zumindest einem Eingabeparameter
- 30-2: Zweiter Verfahrensschritt: Aufnehmen eines Fotos von zumindest einem Körperteil des Fahrradfahrers
- 30-3: Dritter Verfahrensschritt: Bestimmen zumindest eines Körperparameters des Fahrradfahrers
- 30-4: Vierter Verfahrensschritt: Bestimmen jeweils einer Produkteignung einer Mehrzahl von Fahrradprodukten
- 30-5: Fünfter Verfahrensschritt: Bestimmen einer priorisierten Liste der Mehrzahl von Fahrradprodukten
- 30-6: Sechster Verfahrensschritt: Ausgeben einer Auswahl von zumindest einem Fahrradprodukt

- a: Übermitteln eines Fotos von einer App eines Mobilgeräts an eine Steuerung einer externen Datenverarbeitungseinrichtung
- b: Übermitteln zumindest eines Körperparameters von einer Steuerung einer externen Datenverarbeitungseinrichtung an eine App eines Mobilgeräts
- c: Übermitteln von zumindest einem Eingabeparameter und zumindest einem Körperparameter von einer App eines Mobilgeräts an ein Backenduntermodul einer Steuerung einer externen Datenverarbeitungseinrichtung
- d: Übermitteln von zumindest einem Eingabeparameter und zumindest einem Körperparameter von einem Backenduntermodul einer Steuerung einer externen Datenverarbeitungseinrichtung zu einem Datenbankuntermodul einer Steuerung einer externen Datenverarbeitungseinrichtung
- e: Übermitteln von einer priorisierten Liste einer Mehrzahl von Fahrradprodukten von einem Datenbankuntermodul einer Steuerung einer externen Datenverarbeitungseinrichtung zu einem Backenduntermodul einer Steuerung einer externen Datenverarbeitungseinrichtung
- f: Übermitteln von einer priorisierten Liste der Mehrzahl von Fahrradprodukten von einem Backenduntermodul einer Steuerung einer externen Datenverarbeitungseinrichtung zu einer App eines Mobilgeräts

## Patentansprüche

1. Verfahren (30) zum Auswählen zumindest eines Fahrradprodukts aus einer Vielzahl von in einer Datenbank hinterlegten Fahrradprodukten für einen Fahrradfahrer (4), umfassend die folgenden Verfahrensschritte:
Eingeben (30-1) von zumindest einem Eingabeparameter, insbesondere Geschlecht, Fahrradtyp, Fahrhäufigkeit des Fahrradfahrers (4), Körpergewicht des Fahrradfahrers (4), Sitzposition des Fahrradfahrers (4), anatomische Besonderheiten des Fahrradfahrers (4), Körpergröße des Fahrradfahrers (4), Beinstellung des Fahrradfahrers (4), Fußtyp des Fahrradfahrers (4), Schuhgröße des Fahrradfahrers (4), Fußstellung des Fahrradfahrers (4), und/oder eine übliche Dauer einer Fahrradfahrt des Fahrradfahrers (4), durch eine Eingabevorrichtung (9) eines Mobilgeräts (6),
Aufnehmen (30-2) eines Fotos (14) von zumindest einem Körperteil des Fahrradfahrers (4) durch eine Kamera (7) des Mobilgeräts (6),
Bestimmen (30-3) zumindest eines Körperparameters des Fahrradfahrers (4) auf Basis des aufgenommenen Fotos (14) durch eine Steuerung (8-1),
Bestimmen (30-4) jeweils einer Produkteignung einer Mehrzahl von Fahrradprodukten der Vielzahl von in der Datenbank hinterlegten Fahrradprodukten in Abhängigkeit des bestimmten Körperparameters und in Abhängigkeit des zumindest einen eingegebenen Eingabeparameters durch die Steuerung (8-1),
Bestimmen (30-5) einer priorisierten Liste der Mehrzahl von Fahrradprodukten durch die Steuerung (8-1), wobei in der priorisierten Liste die Priorität der jeweiligen Fahrradprodukte mit zunehmender Produkteignung steigt, und
Ausgeben (30-6) einer Auswahl von zumindest einem Fahrradprodukt für den Fahrradfahrer (4) durch das Mobilgerät (6), wobei die Auswahl das zumindest eine Fahrradprodukt der priorisierten Liste der Mehrzahl von Fahrradprodukten umfasst, dessen jeweilige bestimmte Produkteignung die geringste Differenz oder gar keine Differenz zu einer maximalen Produkteignung aufweist, wobei das Verfahren (30) das Auswählen zumindest eines Fahrradsattels aus einer Vielzahl von in einer Datenbank hinterlegten Fahrradsätteln für einen Fahrradfahrer (4) umfasst,
wobei das Eingeben (30-1) des zumindest einen Eingabeparameters das Eingeben von Geschlecht, Fahrradtyp, Fahrhäufigkeit des Fahrradfahrers (4), Körpergewicht des Fahrradfahrers (4), Sitzposition des Fahrradfahrers (4), anatomische Besonderheiten des Fahrradfahrers (4), und/oder eine übliche Dauer einer Fahrradfahrt des Fahrradfahrers (4) umfasst,
wobei das Aufnehmen (30-2) des Fotos (14) das Erfassen eines Gesäßabdrucks (3) des Fahrradfahrers (4) durch eine Erfassungsvorrichtung (2), wobei der Gesäßabdruck (3) jeweils einen Druckbereich (12-1, 12-2) der beiden Sitzknochen des auf der Erfassungsvorrichtung (2) sitzenden Fahrradfahrers (4) umfasst und das Aufnehmen eines Fotos (14) des erfassten Gesäßabdrucks (3) durch die Kamera (7) die Mobilgeräts (6) umfasst,
wobei das Bestimmen (30-3) des zumindest einen Körperparameters des Fahrradfahrers (4) das Bestimmen eines Sitzknochenabstands (16) des Fahrradfahrers (4) auf Basis des aufgenommenen Fotos (14) des erfassten Gesäßabdrucks (3) durch die Steuerung (8-1) umfasst,
wobei das Bestimmen (30-4) der jeweiligen Produkteignung das Bestimmen (30-4) jeweils einer Satteleignung einer Mehrzahl von Fahrradsätteln der Vielzahl von in der Datenbank hinterlegten Fahrradsätteln in Abhängigkeit des bestimmten Sitzknochenabstands (16) und in Abhängigkeit des zumindest einen eingegebenen Eingabeparameters durch die Steuerung (8-1) umfasst,
wobei das Bestimmen (30-5) der priorisierten Liste das Bestimmen (30-5) einer priorisierten Liste der Mehrzahl von Fahrradsätteln durch die Steuerung (8-1) umfasst, wobei in der priorisierten Liste die Priorität der jeweiligen Fahrradsättel mit zunehmender Satteleignung steigt, und
wobei das Ausgeben (30-6) der Auswahl das Ausgeben (30-6) von zumindest einem Fahrradsattel für den Fahrradfahrer (4) durch das Mobilgerät (6) umfasst, wobei die Auswahl den zumindest einen Fahrradsatteln der priorisierten Liste der Mehrzahl von Fahrradsatteln umfasst, dessen jeweilige bestimmte Satteleignung die geringste Differenz oder gar keine Differenz zu einer maximalen Satteleignung aufweist; und
wobei das jeweilige Bestimmen (30-4) der Satteleignung die folgenden Verfahrensschritte umfasst:
Zuordnen jeweils eines numerischen Wertes zu dem bestimmten Sitzknochenabstand (16), und zu dem zumindest einen Eingabeparameter durch die Steuerung (8-1),
Gewichten der jeweils zugeordneten numerischen Werte durch die Steuerung (8-1), um gewichtete numerische Werte zu erhalten, und
Bilden eines Mittelwerts aus der Summe der gewichteten numerischen Werte, um die jeweilige Satteleignung zu erhalten.

2. Verfahren (30) nach Anspruch 1, wobei die Erfassungsvorrichtung (2) eine Grundplatte mit Noppen umfasst, auf welchen ein Blatt Papier (11) angeordnet ist, wobei der Fahrradfahrer (4) während des Erfassens (30-1) des Gesäßabdrucks (3) mit seinem Gesäß auf dem Papier (11) sitzt, und wobei während des Erfassens (30-1) des Gesäßabdrucks (3) die Noppen durch das Papier (11) hindurchgedrückt werden, um die Druckbereiche (12-1, 12-2) der beiden Sitzknochen des Fahrradfahrers (4) auf dem Papier (11) zu erhalten.

3. Verfahren (30) nach Anspruch 2, wobei auf dem Blatt Papier (11) ein umlaufender Rahmen (13) angeordnet ist, und wobei das Bestimmen (30-3) des Sitzknochenabstands (16) das Erkennen und Rektifizieren des aufgenommenen Fotos (14) des Gesäßabdrucks (3) auf Basis des umlaufenden Rahmens (13) durch eine Steuerung (8-1) umfasst.

4. Verfahren (30) nach Anspruch 1, wobei die Erfassungsvorrichtung (2) ein lichttransparentes mit Gel gefülltes Sitzkissen umfasst, wobei der Fahrradfahrer (4) während des Erfassens (30-1) des Gesäßabdrucks (3) mit seinem Gesäß auf dem Sitzkissen sitzt, und wobei während des Erfassens (30-1) des Gesäßabdrucks (3) sich die Verteilung des Gels innerhalb des Sitzkissens verändert, um eine visuelle Darstellung der Druckbereiche (12-1, 12-2) der beiden Sitzknochen des Fahrradfahrers (4) auf dem Sitzkissen zu erhalten.

5. Verfahren (30) nach einem der Ansprüche 1 bis 4, wobei das Bestimmen (30-3) des Sitzknochenabstands (16) des Fahrradfahrers (4) die folgenden Verfahrensschritte umfasst:
Bestimmen eines ersten Mittelpunkts (15-1) eines in dem aufgenommenen Fotos (14) des erfassten Gesäßabdrucks (3) visuell dargestellten ersten Druckbereichs (12-1) eines ersten Sitzknochens des Fahrradfahrers (4) durch die Steuerung (8-1),
Bestimmen eines zweiten Mittelpunkts (15-2) eines in dem aufgenommenen Foto (14) des erfassten Gesäßabdrucks (3) visuell dargestellten zweiten Druckbereichs (12-2) eines zweiten Sitzknochens des Fahrradfahrers (4) durch die Steuerung (8-1), und Bestimmen eines Abstands zwischen dem ersten und zweiten Mittelpunkt (15-1, 15-2) durch die Steuerung (8-1), um den Sitzknochenabstand (16) des Fahrradfahrers (4) zu bestimmen.

6. System (1) zum Auswählen zumindest eines Fahrradsattels aus einer Vielzahl von in einer Datenbank hinterlegten Fahrradsätteln für einen Fahrradfahrer (4), umfassend:
eine Eingabevorrichtung (9) eines Mobilgeräts (6), welche zum Eingeben von zumindest einem Eingabeparameter, welcher Geschlecht, Fahrradtyp, Fahrhäufigkeit des Fahrradfahrers (4), Körpergewicht des Fahrradfahrers (4), Sitzposition des Fahrradfahrers (4), anatomische Besonderheiten des Fahrradfahrers (4), Körpergröße des Fahrradfahrers (4), und/oder eine übliche Dauer einer Fahrradfahrt des Fahrradfahrers (4) umfasst, ausgebildet ist,
eine Erfassungsvorrichtung (2), welche ausgebildet ist, einen Gesäßabdruck (3) des Fahrradfahrers (4) zu erfassen, wobei der Gesäßabdruck (3) jeweils einen Druckbereich (12-1, 12-2) der beiden Sitzknochen des auf der Erfassungsvorrichtung (2) sitzenden Fahrradfahrers (4) umfasst;
eine Kamera (7) des Mobilgeräts (6), welche ausgebildet ist, ein Foto (14) des erfassten Gesäßabdrucks (3) aufzunehmen, und
eine Steuerung (8-1), welche ausgebildet ist, auf Basis des aufgenommenen Fotos (14) einen Sitzknochenabstand (16) des Fahrradfahrers (4) zu bestimmen,
wobei die Steuerung (8-1) ausgebildet ist, jeweils eine Satteleignung einer Mehrzahl von Fahrradsätteln der Vielzahl von in der Datenbank hinterlegten Fahrradsätteln in Abhängigkeit des bestimmten Sitzknochenabstands und in Abhängigkeit des zumindest einen Eingabeparameters zu bestimmen,
wobei die Steuerung (8-1) ausgebildet ist, jeweils einen numerischen Wert zu dem bestimmten Sitzknochenabstand (16), und zu dem zumindest einen Eingabeparameter zuzuordnen,
wobei die Steuerung (8-1) ausgebildet ist, die jeweils zugeordneten numerischen Werte zu gewichten, um gewichtete numerische Werte zu erhalten,
wobei die Steuerung (8-1) ausgebildet ist, einen Mittelwert aus der Summe der gewichteten numerischen Werte zu bilden, um die jeweilige Satteleignung zu erhalten,
wobei die Steuerung (8-1) ausgebildet ist, eine priorisierten Liste der Mehrzahl von Fahrradsätteln zu bestimmen, wobei in der priorisierten Liste die Priorität der jeweiligen Fahrradsättel mit zunehmender Satteleignung steigt,
wobei das Mobilgerät (6) ausgebildet ist, eine Auswahl von zumindest einem Fahrradsattel für den Fahrradfahrer (4) auszugeben, wobei die Auswahl den zumindest einen Fahrradsattel der priorisierten Liste der Mehrzahl von Fahrradsatteln umfasst, dessen jeweilige bestimmte Satteleignung die geringste Differenz oder gar keine Differenz zu einer maximalen Satteleignung aufweist.

## Claims

1. A method (30) for selecting at least one bicycle product from a plurality of bicycle products for a bicyclist (4) stored in a database, with said method comprising the following steps:
entering (30-1) at least one input parameter, particularly gender, bicycle type, riding frequency of the bicyclist (4), body weight of the bicyclist (4), sitting position of the bicyclist (4), anatomical peculiarities of the bicyclist (4), body size of the bicyclist (4), leg position of the bicyclist (4), foot type of the bicyclist (4), shoe size of the bicyclist (4), foot position of the bicyclist (4) and/or a normal duration of a bicycle ride of the bicyclist (4), by means of an input device (9) of a mobile device (6),
capturing (30-2) a photo (14) of at least one body part of the bicyclist (4) by means of a camera (7) of the mobile device (6),
determining (30-3) at least one body parameter of the bicyclist (4) on the basis of the captured photo (14) by means of a control (8-1),
respectively determining (30-4) a product suitability of a multitude of bicycle products of the plurality of bicycle products stored in the database in dependence on the certain body parameter and in dependence on the at least one entered input parameter by means of the control (8-1),
determining (30-5) a prioritized list of the multitude of bicycle products by means of the control (8-1), wherein the priority of the respective bicycle products in the prioritized list increases with increasing product suitability, and
outputting (30-6) a selection of at least one bicycle product for the bicyclist (4) by means of the mobile device (6), wherein the selection comprises the at least one bicycle product of the prioritized list of the multitude of bicycle products, the certain product suitability of which respectively has the smallest difference or no difference at all from a maximum product suitability, wherein the method (30) comprises the selection of at least one bicycle saddle from a plurality of bicycle saddles for the bicyclist (4) stored in the database,
wherein the entry (30-1) of the at least one input parameter comprises the entry of gender, bicycle type, riding frequency of the bicyclist (4), body weight of the bicyclist (4), sitting position of the bicyclist (4), anatomical peculiarities of the bicyclist (4) and/or a normal duration of a bicycle ride of the bicyclist (4),
wherein capturing (30-2) of the photo (14) comprises the acquisition of a buttocks impression (3) of the bicyclist (4) by means of an acquisition device (2), wherein the buttocks impression (3) respectively comprises a pressure region (12-1, 12-2) of the two sit bones of the bicyclist (4) sitting on the acquisition device (2) and a photo (14) of the acquired buttocks impression (3) is captured by means of the camera (7) of the mobile device (6),
wherein the determination (30-3) of the at least one body parameter of the bicyclist (4) comprises the determination of a sit bone spacing (16) of the bicyclist (4) on the basis of the captured photo (14) of the acquired buttocks impression (3) by means of the control (8-1),
wherein the determination (30-4) of the respective product suitability respectively comprises the determination (30-4) of a saddle suitability of a multitude of bicycle saddles of the plurality of bicycle saddles stored in the database in dependence on the certain sit bone spacing (16) and in dependence on the at least one entered input parameter by means of the control (8-1),
wherein the determination (30-5) of the prioritized list comprises the determination (30-5) of a prioritized list of the multitude of bicycle saddles by means of the control (8-1), wherein the priority of the respective bicycle saddles in the prioritized list increases with increasing saddle suitability, and
wherein the output (30-6) of the selection comprises the output (30-6) of at least one bicycle saddle for the bicyclist (4) by means of the mobile device (6), and wherein the selection comprises the at least one bicycle saddle of the prioritized list of the multitude of bicycle saddles, the certain saddle suitability of which respectively has the smallest difference or no difference at all from a maximum saddle suitability,
with the respective determination (30-4) of the saddle suitability comprising the following steps:
respectively assigning a numerical value to the certain sit bone spacing (16) and to the at least one input parameter by means of the control (8-1),
weighting the respectively assigned numerical values by means of the control (8-1) in order to obtain weighted numerical values and
forming an average value from the sum of weighted numerical values in order to obtain the respective saddle suitability.

2. The method (30) according to claim 1, wherein the acquisition device (2) comprises a base plate with knobs, on which a sheet of paper (11) is arranged, wherein the bicyclist (4) sits on the paper (11) with the buttocks during the acquisition (30-1) of the buttocks impression (3), and wherein the knobs are pressed through the paper (11) during the acquisition (30-1) of the buttocks impression (3) in order to obtain the pressure regions (12-1, 12-2) of the two sit bones of the bicyclist (4) on the paper (11).

3. The method (30) according to claim 2, wherein a circumferential frame (13) is arranged on the sheet of paper (11), and wherein the determination (30-3) of the sit bone spacing (16) comprises the recognition and rectification of the captured photo (14) of the buttocks impression (3) on the basis of the circumferential frame (13) by means of a control (8-1).

4. The method (30) according to claim 1, wherein the acquisition device (2) comprises a light-transparent seat cushion that is filled with gel, wherein the bicyclist (4) sits on the seat cushion with the buttocks during the acquisition (30-1) of the buttocks impression (3), and wherein the distribution of the gel within the seat cushion changes during the acquisition (30-1) of the buttocks impression (3) in order to obtain a visual representation of the pressure regions (12-1, 12-2) of the two sit bones of the bicyclist (4) on the seat cushion.

5. The method (30) according to one of claims 1 to 4, wherein the determination (30-3) of the sit bone spacing (16) of the bicyclist (4) comprises the following steps:
determining a first center point (15-1) of a first pressure region (12-1) of a first sit bone of the bicyclist (4), which first pressure region is visually represented in the captured photo (14) of the acquired buttocks impression (3), by means of the control (8-1),
determining a second center point (15-2) of a second pressure region (12-2) of a second sit bone of the bicyclist (4), which second pressure region is visually represented in the captured photo (14) of the acquired buttocks impression (3), by means of the control (8-1), and
determining a spacing between the first and the second center point (15-1, 15-2) by means of the control (8-1) in order to determine the sit bone spacing (16) of the bicyclist (4).

6. A system (1) for selecting at least one bicycle saddle from a plurality of bicycle saddles for a bicyclist (4) stored in a database, with said system comprising:
an input device (9) of a mobile device (6), which is designed for entering at least one input parameter that comprises gender, bicycle type, riding frequency of the bicyclist (4), body weight of the bicyclist (4), sitting position of the bicyclist (4), anatomical peculiarities of the bicyclist (4), body size of the bicyclist (4)
and/or a
normal duration of a bicycle ride of the bicyclist (4),
an acquisition device (2) that is designed for acquiring a buttocks impression (3) of the bicyclist (4), wherein the buttocks impression (3) respectively comprises a pressure region (12-1, 12-2) of the two sit bones of the bicyclist (4) sitting on the acquisition device (2),
a camera (7) of the mobile device (6), which is designed for capturing a photo (14) of the acquired buttocks impression (3), and
a control (8-1) that is designed for determining a sit bone spacing (16) of the bicyclist (4) on the basis of the captured photo (14),
wherein the control (8-1) is designed for respectively determining a saddle suitability of a multitude of bicycle saddles of the plurality of bicycle saddles stored in the database in dependence on the certain sit bone spacing and in dependence on the at least one input parameter,
wherein the control (8-1) is designed for respectively assigning a numerical value to the certain sit bone spacing (16) and to the at least one input parameter,
wherein the control (8-1) is designed for weighting the respectively assigned numerical values in order to obtain weighted numerical values,
wherein the control (8-1) is designed for forming an average value from the sum of weighted numerical values in order to obtain the respective saddle suitability,
wherein the control (8-1) is designed for determining a prioritized list of the multitude of bicycle saddles, wherein the priority of the respective bicycle saddles in the prioritized list increases with increasing saddle suitability,
wherein the mobile device (6) is designed for outputting a selection of at least one bicycle saddle for the bicyclist (4), and wherein the selection comprises the at least one bicycle saddle of the prioritized list of the multitude of bicycle saddles, the certain saddle suitability of which respectively has the smallest difference or no difference at all from a maximum saddle suitability.

## Revendications

1. Procédé (30), destiné à sélectionner au moins un produit pour bicyclette parmi une pluralité de produits pour bicyclettes sauvegardés dans une base de données destinée à un cycliste (4), comprenant les étapes de procédé suivantes, consistant à :
saisir (30-1) au moins un paramètre de saisie, notamment le sexe, le type de bicyclette, la fréquence de déplacement du cycliste (4), le poids du cycliste (4), la position assise du cycliste (4), les particularités anatomiques du cycliste (4), la taille du cycliste (4), la position des jambes du cycliste (4), le type de pied du cycliste (4), la pointure du cycliste (4), la position des pieds du cycliste (4), et / ou une durée usuelle d'un trajet en bicyclette du cycliste (4), à l'aide d'un dispositif de saisie (9) d'un appareil mobile (6),
prendre (30-2) une photo (14) d'au moins une partie du corps du cycliste (4) à l'aide d'un appareil photo (7) de l'appareil mobile (6),
déterminer (30-3) au moins un paramètre corporel du cycliste (4) sur la base de la photo (14) prise, à l'aide d'un système de commande (8-1),
déterminer (30-4) chaque fois une aptitude de produit d'une multiplicité de produits pour bicyclettes parmi la pluralité de produits pour bicyclettes sauvegardés dans la base de données, en fonction du paramètre corporel déterminé et en fonction de l'au moins un paramètre de saisie saisi, à l'aide du système de commande (8-1),
déterminer (30-5) une liste priorisée de la multiplicité de produits pour bicyclettes à l'aide du système de commande (8-1), la priorité des produits pour bicyclettes respectifs croissant dans la liste priorisée au fur et à mesure de la croissance de l'aptitude du produit et
éditer (30-6) une sélection d'au moins un produit pour bicyclette pour le cycliste (4) à l'aide de l'appareil mobile (6), la sélection comprenant l'au moins un produit pour bicyclette de la liste priorisée de la multiplicité de produits pour bicyclettes, dont l'aptitude du produit respectivement déterminée présente la plus faible différence, voire aucune différence par rapport à une aptitude maximale du produit, le procédé (30) comprenant la sélection d'au moins une selle de bicyclette parmi une pluralité de selles de bicyclette sauvegardées dans une base de données pour un cycliste (4),
la saisie (30-1) de l'au moins un paramètre de saisie comprenant la saisie du sexe, du type de bicyclette, de la fréquence de déplacement du cycliste (4), du poids du cycliste (4), de la position assise du cycliste (4), des particularités anatomiques du cycliste (4), et / ou d'une durée usuelle d'un déplacement à bicyclette du cycliste (4),
la prise (30-2) de la photo (14) comprenant l'enregistrement d'une empreinte fessière (3) du cycliste (4) à l'aide d'un dispositif d'enregistrement (2), l'empreinte fessière (3) comprenant chaque fois une zone de pression (12-1, 12-2) des deux ischions du cycliste (4) assis sur le dispositif d'enregistrement (2) et la prise d'une photo (14) comprenant l'empreinte fessière (3) enregistrée à l'aide de l'appareil photo (7) de l'appareil mobile (6),
la détermination (30-3) de l'au moins un paramètre corporel du cycliste (4) comprenant la détermination d'un écart (16) entre les ischions du cycliste (4) sur la base de la photo (14) prise de l'empreinte fessière (3) enregistrée à l'aide du système de commande (8-1),
la détermination (30-4) de l'aptitude respective du produit comprenant la détermination (30-4) de chaque fois une aptitude de la selle parmi une multiplicité de selles de bicyclette de la pluralité des selles de bicyclette sauvegardées dans la base de données, en fonction de l'écart (16) entre les ischions déterminé et en fonction de l'au moins un paramètre de saisie saisi à l'aide du système de commande (8-1),
la détermination (30-5) de la liste priorisée comprenant la détermination (30-5) d'une liste priorisée parmi la multiplicité de selles de bicyclette à l'aide du système de commande (8-1), dans la liste priorisée, la priorité de la selle de bicyclette respective augmentant au fur et à mesure de l'accroissement de l'aptitude de la selle et
l'édition (30-6) de la sélection comprenant l'édition (30-6) d'au moins une selle de bicyclette pour le cycliste (4) à l'aide de l'appareil mobile (6), la sélection comprenant l'au moins une selle de bicyclette de la liste priorisée de la multiplicité de selles de bicyclette, dont l'aptitude de la selle respectivement déterminée présente la plus faible différence, voire aucune différence par rapport à une aptitude maximale de selle ; et
la détermination (30-4) respective de l'aptitude de la selle comprenant les étapes de procédé suivantes, consistant à :
affecter chaque fois une valeur numérique à l'écart (16) entre les ischions déterminé et à l'au moins un paramètre de saisie à l'aide du système de commande (8-1),
pondérer les valeurs numériques respectivement affectées à l'aide du système de commande (8-1), pour obtenir des valeurs numériques pondérées et
créer une valeur moyenne à partir de la somme des valeurs numériques pondérées, pour obtenir l'aptitude respective de la selle.

2. Procédé (30) selon la revendication 1, le dispositif d'enregistrement (2) comprenant une plaque de base pourvue de tétons, sur lesquels est placée une feuille de papier (11), pendant l'enregistrement (30-1) de l'empreinte fessière (3), le cycliste (4) étant assis par son fessier sur le papier (11) et pendant l'enregistrement (30-1) de l'empreinte fessière (3), les tétons étant pressés à travers le papier (11), pour obtenir la zone de pression (12-1, 12-2) des deux ischions du cycliste (4) sur le papier (11).

3. Procédé (30) selon la revendication 2, sur la feuille de papier (11) étant placé un cadre (13) périphérique, et la détermination (30-3) de l'écart (16) entre les ischions comprenant l'identification et la rectification de la photo (14) prise de l'empreinte fessière (3)sur la base du cadre (13) périphérique à l'aide d'un système de commande (8-1).

4. Procédé (30) selon la revendication 1, le dispositif d'enregistrement (2) comprenant un coussin d'assise transparent à la lumière, rempli de gel, pendant l'enregistrement (30-1) de l'empreinte fessière (3), le cycliste (4) étant assis par son fessier sur le coussin d'assise et pendant l'enregistrement (30-1) de l'empreinte fessière (3), la distribution du gel à l'intérieur du coussin d'assise variant, pour obtenir une représentation visuelle de la zone de pression (12-1, 12-2) des deux ischions du cycliste (4) sur le coussin d'assise.

5. Procédé (30) selon l'une quelconque des revendications 1 à 4, la détermination (30-3) de l'écart (16) entre les ischions du cycliste (4) comprenant les étapes de procédé suivantes, consistant à :
déterminer un premier point médian (15-1) d'une première zone de pression (12-1) visuellement représentée dans la photo (14) prise de l'empreinte fessière (3) enregistrée d'un premier ischion du cycliste (4) à l'aide du système de commande (8-1),
déterminer un deuxième point médian (15-2) d'une deuxième zone de pression (12-2) visuellement représentée dans la photo (14) prise de l'empreinte fessière (3) enregistrée d'un deuxième ischion du cycliste (4) à l'aide du système de commande (8-1), et
déterminer un écart entre le premier et le deuxième points médians (15-1, 15-2) à l'aide du système de commande (8-1), pour déterminer l'écart (16) entre les ischions du cycliste (4).

6. Système (1), destiné à sélectionner au moins une selle de bicyclette parmi une pluralité de selles de bicyclette sauvegardées dans une base de données pour un cycliste (4), comprenant :
un dispositif de saisie (9) d'un appareil mobile (6), lequel est conçu pour saisir au moins un paramètre de saisie lequel comprend le sexe, le type de bicyclette, la fréquence de déplacement du cycliste (4), le poids du cycliste (4), la position assise du cycliste (4), les particularités anatomiques du cycliste (4), la taille du cycliste (4), et / ou une durée usuelle d'un trajet en bicyclette du cycliste (4),
un dispositif d'enregistrement (2), lequel est conçu pour enregistrer une empreinte fessière (3) du cycliste (4), l'empreinte fessière (3) comprenant chaque fois une zone de pression (12- 1, 12-2) des deux ischions du cycliste (4) assis sur le dispositif d'enregistrement (2) ;
un appareil photo (7) de l'appareil mobile (6), lequel est conçu pour prendre une photo (14) de l'empreinte fessière (3) enregistrée et
un système de commande (8-1), lequel est conçu pour déterminer sur la base de la photo (14) prise un écart (16) entre les ischions du cycliste (4),
le système de commande (8-1) étant conçu pour déterminer chaque fois une aptitude de selle d'une multiplicité de selles de bicyclette de la pluralité de selles de bicyclette sauvegardée dans la base de données en fonction de l'écart déterminé entre les ischions et en fonction de l'au moins un paramètre de saisie,
le système de commande (8-1) étant conçu pour affecter chaque fois une valeur numérique à l'écart (16) entre les ischions déterminé et à l'au moins un paramètre de saisie,
le système de commande (8-1) étant conçu pour pondérer les valeurs numériques chaque fois affectées, pour obtenir des valeurs numériques pondérées,
le système de commande (8-1) étant conçu pour créer une valeur moyenne de la somme des valeurs numériques pondérées, pour obtenir l'aptitude de selle respective,
le système de commande (8-1) étant conçu pour déterminer une liste priorisée de la multiplicité de selles de bicyclette, dans la liste priorisée, la priorité de la selle de bicyclette respective augmentant au fur et à mesure de l'accroissement de l'aptitude de la selle,
l'appareil mobile (6) étant conçu pour éditer une sélection d'au moins une selle de bicyclette pour le cycliste (4), la sélection comprenant l'au moins une selle de bicyclette de la liste priorisée de la multiplicité de selles de bicyclettes dont l'aptitude respective déterminée présente la plus faible différence, voire aucune différence par rapport à une aptitude maximale de selle.
